# EUROPEAN PATENT SPECIFICATION

(11) **EP 3 993 703 B1**
(45) Date of publication and mention of the grant of the patent: **14.05.2025**
(21) Application number: 20739290.3
(22) Date of filing: 01.07.2020
(51) Int. Cl.: A61M 25/06, A61B 5/15, A61B 5/154

(54) **BLOOD COLLECTABLE PERIPHERAL INTRAVENOUS CATHETER ASSEMBLIES AND RELATED METHODS**
BLUTSAMMELBARE PERIPHERE INTRAVENÖSE KATHETERANORDNUNGEN UND ZUGEHÖRIGE VERFAHREN
ENSEMBLES CATHÉTERS INTRAVEINEUX PÉRIPHÉRIQUES DE COLLECTE DE SANG ET PROCÉDÉS ASSOCIÉS

(30) Priority: 02.07.2019 US 201962869939 P
(43) Date of publication of application: 11.05.2022
(73) Proprietor: B. Braun Melsungen AG, 34212 Melsungen (DE)
(72) Inventor: TEOH, Hui Kuun, 11900 Penang (MY); CHAN, Hwa Loon, Doncaster East, Victoria 3109 (AU)
(74) Representative: Kinkeldey, Daniela
(86) International application number: PCT/EP2020/068569
(87) International publication number: WO 2021/001450

(56) References cited:
- EP-A1- 2 196 227
- EP-A2- 1 430 834
- WO-A1-2014/185905
- US-A- 4 444 203
- US-A1- 2007 060 840

## Description

### FIELD OF ART

The present invention relates to a catheter and holder assembly according to the preamble of claim 1 as well as to a method of assembling a catheter and holder assembly according to claim 12. Such a catheter and holder assembly and such a method are known from US 4 444 203 A.

### BACKGROUND

Catheters are commonly used for a variety of infusion therapies. For example, catheters are used for infusing fluids, such as normal saline solution, various medicaments, and total parenteral nutrition, into a patient, withdrawing blood from a patient, or monitoring various parameters of the patient's vascular system. Following placement of the catheter into the vasculature of a patient, an IV fluid source can be connected to the catheter adapter or catheter hub, opening the blood control valve. Thus connected, fluid from the IV source can begin flow into a patient through the catheter.

For many patients that are admitted to hospitals and undergo catheterization, they will also undergo a separate procedure for blood sampling. As such, the patient will be subject to two separate procedures, which include (a) blood sampling and (b) infusion therapy using IV catheters. The two separate procedures may also require the patient to get more than one needle prick, depending on the devices used in the hospital.

Currently, some IV catheters can allow for attachment of a separate syringe to a cannula hub of the IV catheters. The cannula hub can have a removable blood stopper component at a proximal end of the cannula hub. The syringe can thus be attached to the proximal end of the cannula hub, and the syringe can be used to withdraw blood through the IV catheters. After the syringe is filled with blood, the syringe can then be disconnected from the cannula hub. The blood filled syringe can then be connected to a hypodermic needle, and the needle can be inserted into a vacuum tube, which is commonly referred to as a vacutainer in the relevant industry, for injecting the blood into the vacutainer for blood sampling.

US 4,444,203 discloses intravenous catheter placing and specimen gathering devises.

US 2007/060840 A1 discloses medical devises with needle safety shielding.

WO 2014/185905 A1 discloses vacuum pressure regulators for use during blood collection.

EP 2 196 227 A1 discloses blood drawing devices.

EP 1 430 734 A2 discloses shieldable needle devices with leaf spring driven shield.

### SUMMARY

With two separate procedures for blood sampling and infusion therapy, there can be two sets of hazardous medical waste generated by the procedures. Additionally, where the blood sampling and infusion therapy are separate procedures performed on the patient, the patient can be subjected to at least two needle pricks. Aspects of the present disclosure are directed to a combination device. The combination device may be referred to as a catheter and holder assembly. The catheter and holder assembly can perform multiple medical procedures that otherwise require multiple needle sticks within only a single needle stick.

The catheter and holder assembly can comprise a catheter hub having a catheter tube attached to a blood collection holder having a housing sized and shaped to receive a vacutainer, and wherein a needle having a sharpened distal tip and a sharpened proximal tip passes through both the catheter and the blood collection holder such that the sharpened distal tip is located distal of a catheter tube opening and the sharpened proximal tip is located inside an interior space of the blood collection holder.

According to the present invention there is provided a catheter and holder assembly according to claim 1 comprising: a catheter hub having a catheter tube with a distal opening attached to the catheter hub; a needle comprising a sharpened distal needle tip and a sharpened proximal needle tip and wherein the sharpened distal needle tip projects out the distal opening of the catheter tube; and a blood collection holder having hub with a bore having the needle passing therethrough and a housing having an interior space dimensioned to receive a vacutainer; wherein the catheter hub is in contact with the blood collection holder and the sharpened proximal needle tip is located within the interior space of the housing. Preferred embodiments of the present invention are defined in the dependent claims.

The needle can comprise a notch located proximally of the sharpened distal needle tip.

The blood collection holder can comprise an end wall and a sidewall defining the interior space and the hub can be integrated with the end wall.

The sharpened proximal needle tip can be located in the interior space of the blood collection holder when the catheter assembly is in a ready to use position.

A deformable shield can be retained in the interior space of the blood collection holder, the deformable shield can cover the sharpened proximal needle tip.

The blood collection holder can include a projection that projects into the catheter hub in a ready to use position.

A portion of the needle that extends distally of the blood collection holder and that has the sharpened distal tip for venipuncture can be longer than a portion of the needle that extends into the blood collection holder.

The needle can be fixed to the blood collection holder by means of an adhesive.

The blood collection holder can comprise an interior support column projection proximally from the end wall into the interior space, the interior support column can include a through bore that the needle is inserted through.

The blood collection holder can comprise a distal projection extending distally from an end wall, the distal projection can be configured to fit in an interior of the catheter hub.

The blood collection holder can comprise a distal ring flange arranged radially outward of the distal projection.

The needle can include a bend or curved section corresponding to a bend or curved section in the blood collection holder for fitment.

The bend or curved section of the needle can include a lumen passing therethrough.

The needle can comprise a change in profile located proximally of the sharpened distal needle tip.

A needle guard can be incorporated with the catheter and holder assembly. The needle guard can comprise a surface that is located to a side of the needle in a ready to use position and wherein the surface can be moveable distal of the sharpened distal needle tip in a protective position to cover the sharpened distal needle tip.

A side port and a tubing attached to the side port can be included with the catheter hub.

A valve and a valve opener can be located within the catheter hub.

Aspects of the present disclosure can further include a catheter and holder assembly comprising: a catheter hub comprising a catheter tube; a blood collection holder having a proximal opening sized and shaped to receive a vacutainer; and a needle comprising a sharpened distal needle tip and a sharpened proximal needle tip, the needle being fixed to the blood collection holder; wherein the needle extends through the catheter hub and the catheter tube and the distal needle tip extends distally of an opening of the catheter tube.

Further according to the invention there is provided a method of assembling a catheter and holder assembly according to claim 12. The method can comprise: mounting a needle, comprising a sharpened distal needle tip and a sharpened proximal needle tip, to a blood collection holder; coupling the blood collection holder to a catheter hub comprising a catheter tube so that the needle extends through the catheter hub and the catheter tube and the sharpened distal needle tip is located distally of an end opening of the catheter tube; and wherein the blood collection holder has a body defining an interior space that is dimensioned to receive a vacutainer and the sharpened proximal needle tip is located within the interior space.

Another aspect of the disclosure includes a method for performing blood collection and for peripheral IV access using a single needle and a single needle stick (not being according to the claimed invention).

Another aspect of the disclosure includes a method of using a catheter assembly (not being according to the claimed invention) and wherein the catheter assembly comprises a needle passing through a catheter hub and a catheter tube and wherein the needle comprises a sharpened distal tip located distally of a distal end opening of the catheter tube and a sharpened proximal tip located proximally of a proximal opening of the catheter hub.

The catheter and holder assembly in accordance with further aspects of the disclosure can comprise a blood collection holder or housing, a catheter hub attached to the blood collection holder and having a catheter tube, and wherein a needle comprising a sharpened proximal tip and a sharpened distal tip is located at least partially inside the lumen of the catheter tube and at least partially inside the blood collection holder.

Aspect of the disclosure is further understood to include a catheter and holder assembly comprising a catheter hub having a catheter tube with a distal opening attached to the catheter hub; a needle comprising a sharpened distal needle tip and a sharpened proximal needle tip and wherein the sharpened distal needle tip projects out the distal opening of the catheter tube; and a blood collection holder having hub with a bore having the needle passing therethrough and a housing having an interior space dimensioned to receive a vacutainer; wherein the catheter hub is in contact with the blood collection holder and the sharpened proximal needle tip is located within the interior space of the housing.

A needle usable with a catheter assembly of the disclosure can comprise a notch located proximally of the sharpened distal needle tip.

The blood collection holder can comprise an end wall and a sidewall defining the interior space and the hub is integrated with the end wall.

The needle usable with the catheter assembly of the present embodiment can have two spaced apart sharpened tips. The two sharpened tips can include a distal needle tip and a proximal needle tip. The sharpened proximal needle tip can be in the interior space of the blood collection holder when the catheter assembly is in a ready to use position.

A deformable shield can be retained in the interior space of the blood collection holder, the deformable shield can cover the sharpened proximal needle tip.

The blood collection holder can have a projection that projects into the catheter hub in a ready to use position.

The projection of the blood collection holder can be located distally of the main body, which can be sized and shaped to receive a vacutainer.

A portion of the needle that extends distally of the blood collection holder can be longer than a portion of the needle that extends into the blood collection holder.

The needle can be fixed to the blood collection holder by means of an adhesive.

The blood collection holder can comprise an interior support column projecting proximally from an end wall or distal wall of the blood collection holder and into the interior space of the main body. The interior support column can include a through bore that the needle is inserted through.

The blood collection holder can comprise a distal projection extending distally from an end wall, the distal projection being configured to fit into a proximal opening of the catheter hub. The proximal opening of the catheter hub can be a female Luer, which can be sized and shaped in accordance to ISO Luer standards.

The blood collection holder can comprise a distal ring flange arranged radially outward of the distal projection.

The needle can have a bend located between a proximal end and a distal end. The bend can correspond to a bend formed in the blood collection holder. The two bends, of the needle and of the blood collection holder, can be incorporated for fitment. The bend on the needle can be located closer to the proximal end than the distal end.

The needle can comprise a change in profile located proximally of the sharpened distal needle tip.

The catheter and holder assembly can further comprise a needle guard comprising a surface that is located to a side of the needle in a ready to use position and wherein the surface is moveable distal of the sharpened distal needle tip in a protective position to cover the sharpened distal needle tip.

The catheter hub can include a side port and a tubing attached to the side port.

A valve and a valve opener can be located within the catheter hub. The valve opener can be pushed into the valve, such as a syringe tip or a tip of an IV administration line following successful venipuncture to open the valve. The valve can have a plurality of slits defining a plurality of flaps that can deflect. In an example, there can be three slits and three flaps.

A still further aspect of the disclosure is a catheter and holder assembly comprising: a catheter hub comprising a catheter tube; a blood collection holder having a proximal opening sized and shaped to receive a vacutainer; and a needle comprising a sharpened distal needle tip and a sharpened proximal needle tip, the needle being fixed to the blood collection holder; wherein the needle extends through the catheter hub and the catheter tube and the distal needle tip extends distally of an opening of the catheter tube.

A yet further aspect of the disclosure is a method of assembling a catheter and holder assembly, the method comprising: mounting a needle, comprising a sharpened distal needle tip and a sharpened proximal needle tip, to a blood collection holder; coupling the blood collection holder to a catheter hub comprising a catheter tube so that the needle extends through the catheter hub and the catheter tube and the sharpened distal needle tip is located distally of an end opening of the catheter tube; and wherein the blood collection holder has a body defining an interior space that is dimensioned to receive a vacutainer and the sharpened proximal needle tip is located within the interior space.

The method can further comprise gripping a flange at the proximal end of the blood collection holder.

The method can further comprise abutting the blood collection holder with the catheter hub.

The needle can be fixed to the blood collection holder by means of an adhesive.

The method can further comprise projecting a distal projection extending distally from the end wall of the main body of the blood collection holder into an interior of the catheter hub.

The blood collection holder can comprise a distal ring flange arranged radially outward of the distal projection.

Additionally, it can be advantageous to minimize medical waste by reducing the number of medical devices or components used in the different procedures. It can also be advantageous to minimize the number of needle pricks that a patient must undergo.

Embodiments of the present disclosure may allow for usage of a single needle, such that medical professionals will have one less needle to handle and one less syringe to dispose. By lessening the number of components that the medical professional has to handle, the medical professionals can also be exposed to less risk regarding accidental needle pricks.

An IV catheter assembly, which may more broadly be referred to as a needle assembly or a needle device, can comprise a catheter tube, a catheter hub, a needle, a rubber shield, and a blood collection holder. The catheter hub can have a female Luer at a proximal end for receiving a male Luer in a Luer fit, optionally with external threads.

The catheter tube can be fixed to the catheter hub, such as with a metal bushing or a ferrule.

A needle, which has a change in profile proximal of a distal needle tip, can be inserted through the proximal opening of the catheter hub with the distal needle tip protruding from the distal opening of the catheter tube in a ready to use position. The needle can also have a proximal needle tip at an opposed end of the needle from the distal needle tip. The change in profile can be a crimp, a material buildup, or a sleeve, and can be configured to engage the needle guard and prevent the needle guard from displacing distally off of the needle. In some examples, the change in profile can be omitted and the needle guard can cover the needle tip without using the change in profile.

The needle guard, which is understood to include structural features for guarding the needle tip from unintended needle sticks, can be configured to be removed with the needle following successful venipuncture and the valve and valve actuator can be configured to remain with the catheter hub for controlling fluid flow therethrough, such as to permit fluid communication between a male Luer and the catheter tube. The valve actuator is configured to be pushed distally by a male tip of a medical implement, such as a syringe or an IV connector, into the valve to open the valve for fluid flow. The valve and the valve actuator can be similar to those disclosed in US Pub No. 2018/0214673.

Further information regarding the needle guard is discussed in U.S. Patent No. 8,568,372. Broadly speaking, the needle guard has a surface that is located to the side of the needle shaft in a ready to use position for injection and movable distally of the needle tip in a protective position to block the needle tip from inadvertent needle stick.

In alternative embodiments, the needle guard can embody multiple components that cooperate to block the needle tip from unintended needle sticks. For example, the needle guard can comprise a spring loaded needle carrier having a needle attached thereto. Following successful venipuncture, a release tab can be pressed to release the spring to then move the needle carrier and needle inside a protective barrel to block the needle tip from unintended needle sticks. Further information regarding various aspects of valved catheter assemblies and components thereof are discussed in PCT patent applications PCT/EP2016/069619 and PCT/EP2016/069643 and U.S. Patent No. 9,114,231.

A cannula hub or needle hub can be incorporated at a distal end of the blood collection holder to receive a needle. For example, the needle hub can be part of or can be integrated with the main body of the blood collection holder.

The needle can have a needle tip distal of the needle hub and a second needle tip located inside the interior of the needle holder for puncturing a septum on a vacutainer. In other words, the needle shaft can have a double sharpened needle tips. When assembled with the catheter hub, the present assembly comprises a catheter hub having a catheter tube and a needle with two sharpened needle tips at two opposing ends of the needle shaft. For example, a catheter hub provided herein can include a catheter tube, a needle projecting through the catheter tube, and wherein the needle has a first sharpened needle tip extending out a distal opening of the catheter tube. The needle can have a proximal section extending proximally of the proximal opening of the catheter hub and wherein the proximal section of the needle has a second sharpened needle tip.

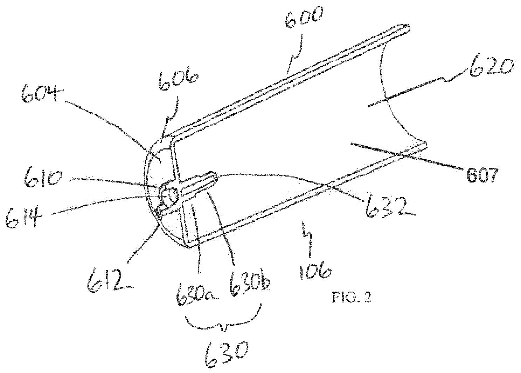

In an embodiment, the needle hub is integrally formed with the body of the blood collection holder. For example, the needle hub can be molded with the body of the holder. Additional detail regarding the blood collection holder and an associated rubber sleeve, also known as a multi-sample Luer adapter or MSLA, are described with respect to FIGs. 2A-3C and elsewhere. **In** addition, the blood collection holder of the present embodiment can be sized and shaped to accept a vacutainer. For example and as further discussed below, the blood collection holder can incorporate a body section having a wall defining an interior sized and shaped to receive a vacutainer.

A rubber shield, a deformable seal or s self-sealing needle sheath, otherwise known as an MSLA, can be attached to the interior of the blood collection holder over a second end of the needle, or the proximal end of the needle. **In** a ready to use position before insertion of a vacutainer into the blood collection holder, the rubber shield can axially surround the proximal end of the needle and cover the proximal needle tip of the needle. The rubber shield can have a main body, a proximal end, and a distal end.

**In** embodiments, the main body of the rubber shield or needle sheath can be generally cylindrical. The self-sealing needle sheath can be made from a rubber or polymer material to allow for deformation in an axial direction of the needle. The open distal end of the self-sealing needle sheath can be sized and shaped to snap fit over a base in the interior of the holder and be assembled therewith for use.

The main body and the distal end of the rubber shield can define an open interior space and an open end that opens into the open interior space. The main body can be defined by a sidewall having a thickness defined between its interior sidewall surface and its exterior sidewall surface. The distal end can have a flange extending radially outward from the main body. The distal end having the flange can have a radial thickness larger than the radial thickness of the main body.

Alternatively, the main body can be formed in a different geometric shape, such as but not limited to a cone, a truncated cone, a pyramid, a truncated pyramid, a prism, a square, or a rectangle. **In** the case of the different geometric shape, the orientation of the geometric shape can be such that the main body can elastically deform in an axial direction of the needle.

The proximal end of the rubber shield can include a covering over the interior of the rubber shield. The proximal end can be considered as a sealed end of the rubber shield. The proximal end can have a domed surface to seal the proximal end of the rubber shield. Alternative shapes, such as a flat surface or conical surface can also be used. A hole or slit may be pre-formed in the surface of the proximal end or the dome for the needle to pass through when the rubber shield is elastically deformed in the axial direction.

If a hole is pre-formed, the hole may be sized such that the surface tension of the blood from the patient prevents leakage or flow past the rubber shield when the rubber shield is in a ready to use position without the needle extending through the surface of the proximal end. Alternatively, a slit, or multiple slits, can be pre-formed in the surface, such that the slit defines two flaps in the surface. When the needle is inserted through the surface of the proximal end, the needle can be inserted in the slit and separate flaps from one another. When the needle is not extending through the surface of the proximal end, then the flaps can seal against one another.

As further described, the blood collection holder can carry the cannula hub at a distal end thereof. The blood collection holder can have a main body that is generally cylindrical. The blood collection holder can have a distal wall at one end of the main body. In some embodiments, the distal wall or end wall can join with the main body with a radius or fillet. From the distal wall, a mating flange can extend in the distal axial direction.

The mating flange can be sized and shaped to project into the interior of the catheter hub when assembled in the ready to use position, similar to a nose end of a needle hub of a standard catheter assembly. The mating flange can embody a cylindrical projection. In some instance, the mating flange and the interior support column can be molded as an independent component, detachable from the end wall. For example, the combination mating flange and interior support column can be screwed on, push off, etc. from the end wall. Optionally, before use or after use, the combination mating flange and interior support column can be separately from the main body.

The mating flange can have a mating flange opening defined by an interior surface of the cylindrical projection. In some embodiments, the mating flange can be a different geometric shape keyed to a corresponding interior shape of the catheter hub in order to prevent rotation of the blood collection holder relative to the catheter hub. In some embodiments, the cylindrical projection of the mating flange can further include a mating projection extending farther in the distal direction from the mating flange. The mating projection can have the same thickness as the thickness of the sidewall of the mating flange.

In embodiments, the mating projection can have a surface configured to push the needle guard into engagement with the interior engagement projection formed with the catheter hub when the blood collection holder is mated with the catheter hub. This allows the needle guard to be held in place in the ready to use position and during retraction of the needle following successful venipuncture, to prevent early activation.

At the opposite end, such as the proximal end of the main body, there can be an opening for receiving a vacutainer into the interior space of the blood collection holder. Near or at the proximal end of the main body, a handle or gripping pad can project radially outward from the main body. The handle can be substantially rectangular in shape. In some embodiments, the blood collection holder can have one handle extending radially outward from the main body.

In other embodiments, the blood collection holder can have two or more handles extending radially outward from the main body. With two or more handles, the handles can be spaced equidistantly around the main body or spaced to be ergonomically comfortable for one handed gripping of the blood collection holder.

A metal bushing can be configured to wedge the proximal end of the catheter tube against the interior wall surfaces of the catheter hub to retain the catheter tube to the catheter hub. Interiorly of the catheter hub, a septum or valve, an actuator or valve opener and a needle guard, such as a safety shield or tip protector, are provided. The catheter hub can be made from a single hub body as shown or from two or more hub bodies assembled together.

The septum or valve can include at least one slit, defining elastically deformable flaps of the valve. In an example, the valve has more than one slit and more than one flaps, such as three slits and three valve flaps. The needle can be inserted through the at least one slit such that the elastic flaps can be expanded by the hollow needle and closed when the needle is removed. The valve can be sized and shaped for multiple use by cooperating with the valve opener to open the one or more slits of the valve for fluid flow and closing upon removal of a male Luer tip that was placed into the catheter hub to push the valve opener in the distal direction. In some examples, the flaps can remain open and engaged with a valve opener in a one-time activation configuration. The number of slits defining the number of flaps can vary, ranging from one slit to four or more slits.

The catheter hub can have a proximal end with an opening. The proximal end can also be provided with a female Luer fitting with exterior threads, forming a female threaded Luer. In some examples, the threads can be omitted and the proximal opening can function as a female Luer slip.

A catheter hub provided herein can include a pair of wings. Embodiments of the catheter hub can also include embodiments without wings.

In the interior space of the blood collection holder, an interior support column can extend proximally into the interior space from the distal wall. The interior support column can be generally cylindrical or conical in shape, extending from the axial center of the blood collection holder. The interior support column can include a distal section and a proximal section. The distal section, which is closer to the distal wall or end wall, can have a first outer diameter that is a larger diameter than a second outer diameter of the proximal section.

The transition of the outer diameter from the distal section to the proximal section can be stepped or tapered. The first outer diameter of the distal section can be sized to correspond to the interior diameter of the rubber shield for fixing the rubber shield to the distal section. As the rubber shield can be elastically deformable and stretchable, the first outer diameter can be larger than the interior diameter of the rubber shield when the rubber shield is in its relaxed state. Accordingly, the rubber shield can be fitted over the distal section to fix the rubber shield to the distal section.

In some embodiments, the proximal section can have a second outer diameter than the smaller interior diameter of the rubber shield. The smaller second outer diameter of the proximal section can allow for space to accommodate the elastic deformation of the rubber shield when the rubber shield compresses in the axial direction towards the distal wall.

A bore can extend through the interior support column and through to the mating flange opening. The bore can be sized and shaped to accommodate the needle. The bore can be sized for an interference press fit with the needle and/or can include a gap, or glue well, sufficient for adhesive to fix the needle to the bore.

The blood collection holder can be mated with the needle and the rubber shield. The rubber shield can mate with the interior support column, extending proximally into the interior space from the distal wall or end wall. The interior support column can include a distal section and a proximal section. The distal section of the interior support column that is closer to the distal wall can have a first outer diameter that is a larger diameter than a second outer diameter of the proximal section.

In some embodiments, the rubber shield can be elastically deformable and stretchable, such that the first outer diameter of the distal section can be larger than the interior diameter of the rubber shield when the rubber shield is in its relaxed state. Accordingly, the rubber shield can be fitted over the distal section to fix the rubber shield to the distal section. In other embodiments, the rubber shield can have a rigid structure near its distal end and have an interference fit with the first outer diameter of the distal section.

The rubber shield can be fitted such that the flange of the rubber shield abuts the distal wall. Additionally, the rubber shield can be secured to the distal section by way of a mechanical means such as a clip or by adhesive. In some embodiments, the rubber shield can have a relaxed fitment over the distal section and be secured by the clip or adhesive. This may facilitate easier assembly.

The transition of the outer diameter from the distal section to the proximal section can be stepped or tapered. Proximal to the distal section, the proximal section of the interior support column can have a smaller second outer diameter than the first outer diameter of the distal section. The smaller second outer diameter of the proximal section can allow for space to accommodate the elastic deformation of the rubber shield when the rubber shield compresses in the axial direction towards the distal wall due to insertion of a vacutainer into the blood collection holder.

The proximal needle tip can be located inside the interior of the rubber shield. As such, the proximal needle tip is located at an intermediary position between the interior support column and the proximal end of the rubber shield. This way, the rubber shield surrounds the proximal needle tip, separating the proximal needle tip from the remainder of the interior space of the blood collection holder.

In this way, in the ready to use position, the rubber shield can act as a sanitary shield to prevent contamination of the proximal needle tip and to seal the needle from blood flashback during replacement of the vacutainer with a new or different vacutainer. When a vacutainer is inserted into the interior space of the blood collection holder, the vacutainer can compress and elastically deform the proximal end of the rubber shield axially towards the distal wall. The proximal end of the rubber shield can be elastically deformed past the proximal needle tip, such that the proximal needle tip extends proximally of the proximal end of the rubber shield and into the vacutainer.

At the distal end of the bore, the mating flange can extend distally of the distal wall of the blood collection holder. The cylindrical projection of the mating flange can further include a mating projection extending farther in the distal direction from the mating flange. The mating projection can include a primary proximal portion, an arcuate section extending farther in the distal direction from the cylindrical projection that is the mating flange.

The mating projection can have a distal key portion extending distally from the primary proximal portion. The distal key portion can have a similar outer arcuate shape as the primary proximal portion and the mating flange. The distal key portion can have a radially inner flat portion to set the needle guard in the catheter hub.

In the assembled, ready to use position of the attached configuration of the blood collectable peripheral intravenous catheter assembly or catheter and holder assembly, the catheter hub abuts the distally facing surface of the distal wall of the blood collection holder. The needle extends through the catheter hub and the catheter tube, with a needle tip extending distally of the catheter tube opening.

In the ready to use position, the catheter hub abuts the distally facing surface of the distal wall of the blood collection holder with the mating projection and the mating flange of the blood collection holder projecting into the interior of the catheter hub.

The needle extends through the catheter hub and the catheter tube, thereby extending through the valve and the valve opener, which are located in the interior of the catheter hub. As the needle provides a through connection from the distal needle tip to the proximal needle tip, the needle provides a through connection from the distal needle tip to the space between the proximal needle tip and the rubber shield in the interior space of the blood collection holder.

In the ready to use position, the needle guard is positioned along the needle. For example, the needle can project through an opening on a proximal wall of the needle guard. Upon retraction of the needle, the crimp or change in profile formed with the needle can move proximally of two distal walls at an end of two arms of the needle guard. As the needle moves further proximally, the crimp or change in profile can engage the opening on the proximal wall to retain the needle guard on the needle and not fall off distally of the needle tip.

The needle guard, which is understood to include structural features for guarding the needle tip from unintended needle sticks, is configured to be removed with the needle following successful venipuncture. The valve and valve actuator are configured to remain with the catheter hub, such as inside the interior of the catheter hub, for controlling fluid flow therethrough.

In an exemplary embodiment, as further described in the reference of U.S. Patent No. 8,568,372, the change in profile of the needle can be configured to engage with an outer circumference defining a proximal opening on the rear wall or proximal wall of the needle guard so that the needle guard can be removed from the catheter hub with the needle. The proximal wall of the needle guard can be slidably located between the location of the change in profile and the proximal end of the needle. Thereby, the proximal wall of the needle guard can be prevented from passing the change in profile and from becoming separated from the needle.

The fitment of the needle guard in the catheter hub can also serve to prevent rotation of the needle guard. The needle guard can be fitted in the catheter hub such that rotation of the needle guard relative to the catheter hub can be prevented or minimized.

The radially inner flat portion of the distal key portion of the blood collection holder can contact a portion of the needle guard. In an exemplary embodiment, the needle guard can have a corresponding proximal flat surface or edge for contact with the flat portion of the distal key portion.

The catheter hub can be spaced from the needle and the needle guard covering the needle tip can resemble a configuration in which activation and separation occur following successful venipuncture. In the actuated position following initial placement of the catheter into the vein for blood sampling then subsequently advanced for peripheral venous access, the blood collection holder is separated from the catheter hub. The needle is withdrawn proximally from the catheter tube and the catheter hub following peripheral venous access.

When the needle is withdrawn from the catheter hub, the needle guard stays coupled to the needle and is withdrawn from the catheter hub with the needle. The needle guard can cover the distal needle tip to prevent accidental needle stick when the needle is withdrawn from the catheter hub and the crimp or material build up on the needle near the distal needle tip prevents the needle guard from displacing distally off of the needle.

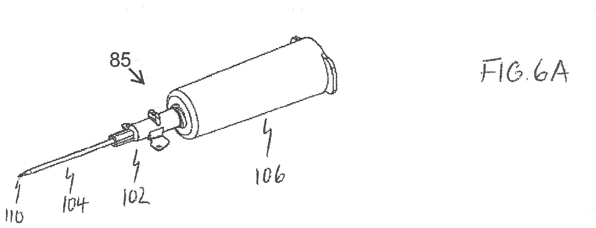

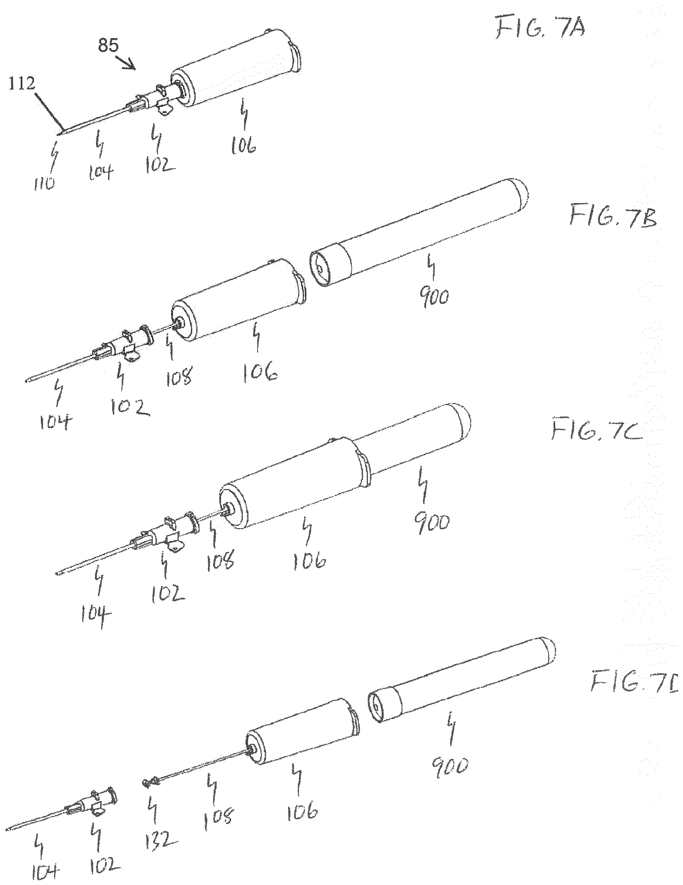

At least two processes or methods of usage of the catheter and holder assembly of the present disclosure can be envisioned (not being according to the claimed invention). FIGs. 6A-6D illustrate a first process for usage wherein blood sampling is performed while the blood collection holder is mated to the catheter hub. FIGs. 7A-7C illustrate a second process for usage wherein the catheter tube is advanced into a patient's vein, thereby separating the blood collection holder from the catheter hub, before performing blood sampling.

In the first process for usage of the catheter and holder assembly, blood sampling can be performed with the blood collection holder mated to the catheter hub, such that the distal needle tip of the needle extends distally of the distal opening of the catheter tube during blood sampling.

In the ready to use position with the catheter and holder assembly, which has a housing with a proximal opening sized and shaped to receive a vacutainer mated to the catheter hub, as described elsewhere herein, the distal needle tip extends distally of a distal opening of the catheter tube such that the distal needle tip can be used to access the vein like a conventional blood collection procedure. Upon successful accessing the vein, the distal needle tip and the distal end of the catheter tube are located inside the vein, thereby allowing needle to provide a fluid communication between the vein and the interior space of the blood collection holder, and particularly to a proximal needle section of the needle having a second sharpened needle tip that is covered by a rubber sheath or shield to limit or prevent blood flashback into the interior space of the holder.

The medical professional can then hold the catheter assembly with one hand in a steady manner to avoid movement of the catheter assembly relative to the vein to enable blood collection. In some embodiments, the catheter hub can be provided with a pair of wings to facilitate securement of the catheter hub to a patient following venipuncture. The pair of wings can be flanges extending radially outward from the catheter hub and flexible to contact the patient's skin.

The vacutainer can be inserted while the blood collection holder, such as the distal end of the holder, is still in contact with the catheter hub. The insertion of the vacutainer can axially deform the rubber shield in the interior space of the blood collection holder such that the rubber shield is deformed distally past the proximal needle tip by the septum on the vacutainer. As the vacutainer is fully inserted, the proximal needle tip can pierce the septum of the vacutainer and be inserted into the vacutainer, such that the interior of the vacutainer can be in a fluid communication with the needle lumen.

With the fluid connection through the needle, blood sampling with the vacutainer can thus be performed while the blood collection holder stays mated to the catheter hub.

Upon removal of the vacutainer after successful blood sampling, the rubber shield can return to its ready to use position, thereby covering the proximal needle tip of the two-sharpened-tip-needle and preventing blood leakage through the proximal needle tip. The elastic deformation of the rubber shield allows for repeated blood sampling by serially inserting additional vacutainers into the interior space of the holder while also preventing blood leakage when each vacutainer is removed from the blood collection holder.

After the last of the vacutainers is removed from the holder, the needle and the holder can retract proximally away from the catheter hub. After successful blood sampling has been completed, the medical professional can advance the catheter hub and the catheter tube to place the catheter tube into the vein as normally done per peripheral IV catheter (PIVC) procedures. In this way, the medical professional can maintain the position of the blood collection holder, and thereby the needle, relative to the vein, and advance the catheter tube into the vein by moving the catheter hub in the distal direction.

Upon advancing of the catheter hub and the catheter tube, the medical professional can fully remove the blood collection holder and the needle attached to the holder from the catheter hub by retracting the blood collection holder in the proximal direction relative to the catheter hub.

Optionally, the last of the vacutainers used with the main body of the blood collection holder can remain with the blood collection holder while the blood collection holder is retracted away from the catheter hub. Then the last of the vacutainer can be removed from the blood collection holder before or after separation of the holder from the catheter hub.

On withdrawal of the hollow needle from the catheter hub following successful venipuncture, a change in profile provided near the distal needle tip and having the form of a radial projection on the hollow needle, such as by crimping, engages with the safety shield so that the safety shield can be removed from the catheter hub with the needle. The safety shield can then act as a shield or cover the distal needle tip of the needle as it is withdrawn from the catheter hub.

In an exemplary embodiment, the safety shield can have two arms that can move to cover the distal needle tip. As the distal needle tip moves proximally of distal walls of the two arms, the two arms are no longer biased by the needle and can move, such as spring or deflect radially to disengage from the interior of the catheter hub. As the arms of the safety shield move radially, the arms, or the distal walls of the arms, can cover the needle tip to prevent unintended needle sticks. That is, the distal walls can move from a position to the side of the needle tip to a position distal of the needle tip to prevent unintended needle sticks.

In some examples, the needle shield can have only one arm and one distal wall at an end of the one arm. In other examples, the change in profile can include a sleeve, a notch, or a material buildup on the shaft of the needle. Using a notched needle near the distal tip allows blood flashback to be viewed in the annular space between the needle and the catheter tube. Further information regarding the safety shield is discussed in U.S. Pat. No. 7,736,339.

Following removal of the needle after successful catheterization, the one or more flaps of the valve located inside the catheter hub, due to their elastic properties, close the one or more slits through the depth of the valve disk or disc so that no blood or substantially no blood can flow from the catheter tube into the proximal chamber of the catheter hub, and out through the proximal opening of the catheter hub. In this way, the valve prevents further blood spill or flow.

Accordingly, the process described herein can provide for both blood sampling and catheterization for IV infusion with only one needle stick using the catheter and holder assembly of the present invention. In comparison, prior art procedures can involve a first needle stick using a blood collection needle to collect blood and then after the blood collection and the blood collection needle is removed, the procedures can involve a second needle stick for PIVC access for IV infusion. In the present embodiment, the blood collection holder has an integrated needle hub for securing a needle having two sharpened needle tips, a distal needle tip and a proximal needle tip. The blood collection holder further comprises a housing or main body attached to the needle hub, or said differently - the needle hub is attached to a main body of a blood collection holder. The housing being generally cylindrical and having an interior that is sized and shaped to receive a vacutainer. The proximal needle tip can be positioned in the interior of the housing for puncturing a septum on the vacutainer to provide fluidic communication between the distal needle tip, the needle lumen, the proximal needle tip, and the interior of the vacutainer, when the needle is not shielded by the rubber sheath or shield.

A second process for usage of the catheter and holder assembly of the present invention includes performing blood sampling after the medical professional has advanced the catheter tube into the patient, similar to normal PIVC access, but before fully removing the needle from the catheter tube lumen. Thus, before the needle that is attached to the blood collection holder is completely removed from the catheter tube, blood collection can be performed after the blood collection holder has separated from the catheter hub.

In the ready to use position with the blood collection holder mated to the catheter hub, the distal needle tip extends distally of a distal opening of the catheter tube such that the distal needle tip can be used to access the vein. The distal needle tip is then retracted proximally of the distal opening of the catheter tube as the catheter tube is further advanced into the vein. At this time, primary and secondary flashbacks can be confirmed. While the catheter tube is further located inside the vein and the needle is retracted in the proximal direction but not completely out of the lumen of the catheter tube, the needle maintains fluid communication between the vein and the interior space of the blood collection holder, such as through to the proximal needle tip.

The medical professional can then either partially or fully advance the catheter tube into the patient's vein prior to blood sampling. Optionally, a tourniquet may be used with the present catheter and holder assembly as conventionally practiced. Where not permitted, such as under restrictions by certain local rules, a tourniquet may not be used and if used where not permitted, the first bottle or vacutainer may need to be discarded.

The medical professional can then hold the catheter assembly with one hand sufficiently steady to prevent movement of the catheter assembly relative to the vein. In some embodiments, the catheter hub can be provided with a pair of wings to facilitate securement of the catheter hub to a patient following venipuncture. The pair of wings can be flanges extending radially outward from the catheter hub and flexible to contact the patient's skin.

With the advancement of the catheter hub to advance the catheter tube into the vein and proximal retraction of the needle during the catheter tube advancement, the blood collection holder and the catheter hub can be separated, or physically spaced, along the axial direction of the needle while still coupled by the needle and the lumen of the catheter tube. In the ready to sample position, the needle, which is fixed to the blood collection holder, can still be in the vein of the patient or can be recessed from the distal opening of the catheter tube and located in the lumen of the catheter tube such that the needle, which is in fluid communication with the lumen of the catheter tube, provides a fluid connection between the vein and the proximal needle tip.

In practice, the advancement of the catheter hub with the distal needle tip recessed or retracted in the proximal direction from the distal opening of the catheter tube can serve as a safeguard against accidental advancement of the needle in the patient.

The vacutainer can be inserted into the blood collection holder after the blood collection holder retracts from the catheter hub but wherein the needle is still in fluid communication with the catheter tube. The insertion of the vacutainer can axially deform the rubber shield or MSLA in the interior space of the blood collection holder such that the rubber shield is deformed distally past the proximal needle tip to expose the needle tip. As the vacutainer is fully inserted, the proximal needle tip can pierce the septum and be inserted into the vacutainer, such that the vacutainer can be in a fluid connection with the proximal needle tip.

With fluid connection between the vacutainer and the needle, blood sampling can thus be performed while the blood collection holder stays mated to the catheter hub via the needle.

Upon removal of the vacutainer after successful blood sampling, the rubber shield can return to its ready to use position, thereby covering the proximal needle tip and preventing blood leakage through the needle and out the proximal opening of the catheter hub. The elastic deformation of the rubber shield allows for repeated blood sampling with a plurality of vacutainers, while also preventing blood leakage when each of the vacutainers is removed from the blood collection holder.

After successful blood sampling has been completed, the medical professional can then remove the blood collection holder and the attached needle from the catheter hub. The medical professional can also fully remove the blood collection holder and the needle from the catheter hub by retracting the blood collection holder axially in the proximal direction relative to the catheter hub.

On withdrawal of the hollow needle from the catheter hub following successful venipuncture, a change in profile provided near the distal needle tip and having the form of a radial projection on the hollow needle, such as by crimping, engages with the safety shield so that the safety shield can be removed from the catheter hub with the needle, as previously described elsewhere herein. The safety shield can then act as a shield or cover the distal needle tip of the needle as it is withdrawn from the catheter hub.

Following removal of the needle after successful catheterization, the one or more flaps of the valve, due to their elastic properties, close the one or more slits through the depth of the valve disk or disc so that no blood or substantially no blood can flow out through the proximal opening of the catheter hub. In this way, the catheter hub can prevent further blood loss or flow.

Accordingly, using a catheter and holder assembly of the present invention, the process described can provide for blood sampling and catheterization with only one needle stick. In contrast, prior art devices require a first needle stick with a blood sampling device to obtain blood samples via vacutainers. The blood sampling device is then removed from the patient after the desired number of samples, such as tubes, have been taken. A second needle stick with a catheter assembly is then carried out to obtain peripheral IV access.

After successful venipuncture, the catheter hub can then connect with a drip line. However, the catheter and holder in accordance with aspects of the present invention can perform both the blood sampling function and the peripheral IV access with only a single needle stick. The present assembly thus reduces waste, by utilizing fewer components and at least one less needle, and reduces the number of needle sticks to one, which can minimize needle stick injuries and potential stress on the patient from the possibly receiving more than one needle sticks.

In an alternative embodiment, a protrusion cover around the mating flange, with a gap or space between the two, can be included. As previously described, the mating flange is configured project into the catheter hub. A stopper projection can be provided on the inside surface of the protrusion cover. The protrusion cover can be sized and shaped to project around the exterior of the catheter hub, at the proximal end of the catheter hub, and the mating flange is configured to project into the proximal opening of the catheter hub. The protrusion cover can prevent accidental or early separation of the catheter hub from the blood collection holder during cannulation and blood sampling. Accidental separation of the catheter hub and the blood collection holder can be unnerving for a medical professional and creates a risk of inadvertent needle movement while the medical professional adjusts to the relative movement of the catheter hub.

In order to prevent accidental movement, the blood collection holder can have a protrusion cover, which can be a cylindrical projection extending distally from the distal wall of the blood collection holder. The cylindrical projection can have a wall structure that is spaced radially outward from the mating flange, such that the protrusion cover and the mating flange are concentric with each other, and the protrusion cover extends distally from the distal wall to cover a proximal portion of the catheter hub when mated with the blood collection holder.

In an example, the exterior surface of the protrusion cover can also be profiled, such as include detents, engagement features, interference surface, to engage a protective sheath that placed over the catheter assembly during packaging and shipping, if the catheter assembly and blood collection holder were packaged as a single assembly.

On the interior surface of the protrusion cover, a stopper projection can extend radially inwardly from the interior surface. The stopper projection can be a single raised lip or bump or an annular protrusion that is formed around the interior circumference of the protrusion cover. Optionally, the stopper projection can be non-continuous and formed around the interior circumference by providing a plurality of spaced apart individual bumps. The stopper projection is configured to contact a rear exterior feature or surface of the catheter hub, such as the exterior threads of the catheter hub, to restrict relative movement.

In yet another alternative embodiment of the blood collection holder, a protrusion cover formed around the mating flange and provided on the interior surface with two stopper projections having a space or gap in between. The cover protrusion of the present embodiment can include a second stopper projection located axially farther from the distal wall, or distally spaced from the distal wall, of the blood collection holder and spaced from the first stopper projection. Like the first stopper projection, the second stopper projection can be a single raised lip or bump or an annular protrusion that is formed around the interior circumference of the protrusion cover. Optionally, the second stopper projection can be non-continuous and formed around the interior circumference by providing a plurality of spaced apart individual bumps. The second stopper projection can be aligned radially with the first stopper projection. Alternatively, the second stopper projection can be offset radially along the circumference of the interior of the cover protrusion.

With the second stopper projection, even if the catheter hub is accidentally moved past the first stopper projection, the catheter hub can be retained by the second stopper projection. The second stopper projection can be viewed as a backup to the first stopper projection. The present embodiment can be viewed as a blood collection holder having two separation stops, or one stop with one backup stop.

The first stopper projection and the second stopper projection can be the same size and the same shape. Alternatively, the first stopper projection and the second stopper projection can be of different sizes, such that different levels of force can be applied to the catheter hub to require different level of forces to overcome the two stopper projections. For example, the second stopper projection can require a higher separation force than the first stopper projection by incorporating a more inwardly surface structure or lip from the interior surface of the protrusion cover than the first stopper projection.

Additional stopper projections, such as more than two rows of stopper projections, can be provided to provide additional blocks from separation of the catheter hub from the blood collection holder. Additionally, alternative forms of stopper projections can be used. Although the exemplary embodiments use domed, annular lips, protrusions, or bump stopper projections, the stopper projections could alternatively be of another geometric shape, such as a rectangle, different shapes, detents, relatively sharp gripping structures like barbs, etc., or be an arcuate flange on the interior of the cover protrusion. The stopper projections can be of different shapes from one another.

In yet another alternative embodiment, the body of catheter hub is provided with side fluid port and tubing, also known as an integrated catheter hub. The integrated catheter hub can include a side fluid port extending obliquely from the generally cylindrical body of the catheter hub.

A tubing can be connected to the side fluid port at its first end and the opposite end of the tubing can be connected to a fluid adapter, such as a male or a female needleless valve, which a syringe without a needle can attach and infuse fluids, such as medication. The tubing can have a lumen for fluid flow and provide a fluid connection between the catheter hub 102 through the side fluid port and an external adapter.

The external adapter can be any type of conventionally known adapter, such as a Luer connector, a needleless connector, or a conventional vent plug. Blood flow can be stopped by a seal or septum located at the proximal end of the catheter hub, proximal of where the side fluid port opens into the catheter hub so as to not interfere with fluid flow through the side fluid port. IV fluid or other medicaments can flow into the catheter hub via connection from the external fluid adapter through the tubing and through the side fluid port.

Additionally, the catheter hub of the present embodiment can have a pair of wings projecting from the body of the catheter hub to aid in securement to the patient, such as an adhesive dressing.

Alternative blood collection holders can include a cover protrusion or stopper projections for use with the integrated catheter hub. The side fluid port and the tubing can be arranged such that they do not interfere with the fitment of the protrusion cover when mating the blood collection holder and the integrated catheter hub.

The operating process for a catheter assembly using an integrated catheter hub can be the same as that described elsewhere herein.

In yet another alternative needle, the shaft of the needle can have a curve feature or curved section for fitment with a corresponding shaped groove in the blood collection holder. The needle can have the curve feature or bend at an intermediary position between the distal needle tip and the proximal needle tip. The curve feature can be a simple C-shaped bend, such that the distal needle tip and a distal section of the needle shaft are axially aligned with the proximal needle tip and a proximal section of the needle shaft. Alternatively, the bend can be a different shape, such as an S-shape, or a complex shape.

The bore of the blood collection holder can likewise have a corresponding slot or shape for receiving or accommodating the curved section or bend of the needle. The needle can be fitted and snapped into the corresponding slot, or corresponding curved feature, of the blood collection holder. In some embodiments, the needle can be forced into the corresponding slot by axial insertion.

In some embodiments, the blood collection holder, at least at the needle hub section of the holder, can be a two part construction with division along the bore, such that the needle can be snapped into the corresponding slot prior to assembly of the blood collection holder. The corresponding slot can have a corresponding profile as the bend section of the needle and a slight undercut feature so that a slight force is needed to push the needle into the slot.

The combination of the corresponding slot and the bend feature can adequately secure the cannula mechanically, without needing additional adhesive. Alternatively, adhesive can be used or other bonding technique such as ultrasonic welding can be used to bond the needle with the blood collection holder even when using the bend feature and the corresponding shaped slot.

Methods of making and of using needle devices and assemblies and components thereof are not understood to be within the scope of the claimed invention.

### BRIEF DESCRIPTION OF THE DRAWINGS

These and other features and advantages of the present devices, systems, and methods will These and other features and advantages of the present devices, systems, and methods will become appreciated as the same becomes better understood with reference to the specification, claims and appended drawings wherein:
FIG. 1A is an exploded view of a valved catheter assembly with a blood collection holder.
FIG. 1B shows a cross-sectional view of the catheter hub taken along the axial direction with a catheter tube and a bushing, among other components.
FIG. 2 shows a cross sectional view of the embodiment of the blood collection holder.
FIGs. 3A and 3B show a needle and the blood collection holder in an attached or assembled configuration and a cross-sectional view thereof.
FIGs. 4A and 4B show the catheter hub, the needle, and the blood collection holder in an attached or assembled configuration.
FIG. 5 shows an exploded cross-sectional view of an exemplary embodiment of the catheter assembly and a vacutainer.
FIGs. 6A-6D show different stages of an embodiment of the catheter assembly used for blood sampling and for catheterization.
FIGs. 7A-7D show a second process for usage wherein blood sampling is performed after the medical professional has advanced the catheter tube into the patient
FIGs. 8A and 8B show an alternative embodiment of the blood collection holder with a cover protrusion and a stopper projection.
FIGs. 9A and 9B show another alternative embodiment of the blood collection holder with a cover protrusion and two stopper projections.
FIGs. 10A and 10B show an alternative embodiment of the catheter hub wherein the catheter hub is an integrated catheter hub.
FIGs. 11A and 11B show an embodiment of a curved needle for fixing to the blood collection holder.

### DETAILED DESCRIPTION

The detailed description set forth below in connection with the appended drawings is intended as a description of the presently preferred embodiments of blood collection holders and IV catheters provided in accordance with aspects of the present devices, systems, and methods and is not intended to represent the only forms in which the present devices, systems, and methods may be constructed or utilized. The description sets forth the features and the steps for constructing and using the embodiments of the present devices, systems, and methods in connection with the illustrated embodiments. It is to be understood, however, that the same or equivalent functions and structures may be accomplished by different embodiments that are also intended to be encompassed within the scope of the present disclosure. As denoted elsewhere herein, like element numbers are intended to indicate like or similar elements or features.

With two separate procedures for blood sampling and infusion therapy, there can be two sets of hazardous medical waste generated by the procedures. Additionally, where the blood sampling and infusion therapy are separate procedures performed on the patient, the patient can be subjected to at least two needle pricks.

Even in the case where a syringe attached to a cannula hub of a PIVC is used, this requires the operator to attach and detach components. The need to add a hypodermic needle to the syringe in order to fill a vacutainer further adds the potential of an accidental needle prick when connecting the hypodermic needle to the blood-filled syringe and handling the needle tipped syringe. Minimizing the risk of accidental needle sticks for medical professionals is important because of the prevalence of potentially fatal infectious diseases, such as, for example, Acquired Immune Deficiency Syndrome (AIDS) and hepatitis, that can be transmitted by the exchange of bodily fluids through inadvertent wounds caused by accidental needle tip pricks from handling used needles.

Additionally, it can be advantageous to minimize medical waste by reducing the number of medical devices or components used in the different procedures. It can also be advantageous to minimize the number of needle pricks that a patient must undergo.

Embodiments of the present disclosure may allow for usage of a single needle, such that medical professionals will have one less needle to handle and one less syringe to dispose of. By lessening the number of components that the medical professional has to handle, the medical professionals can also be exposed to less risk regarding accidental needle pricks.

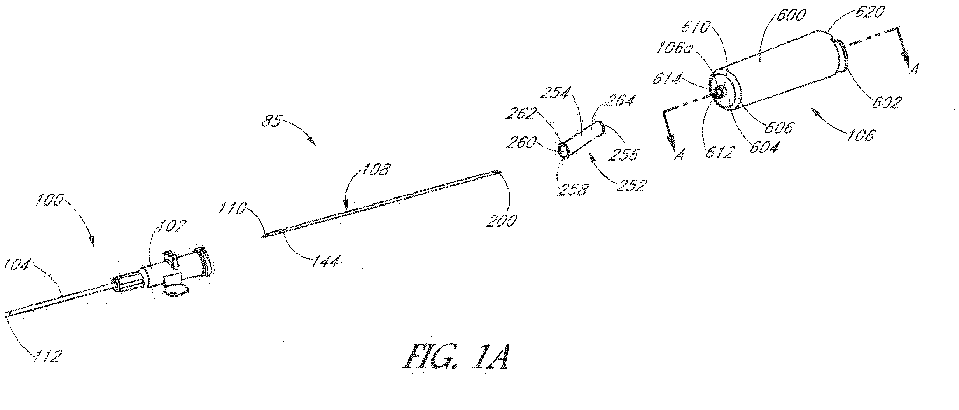

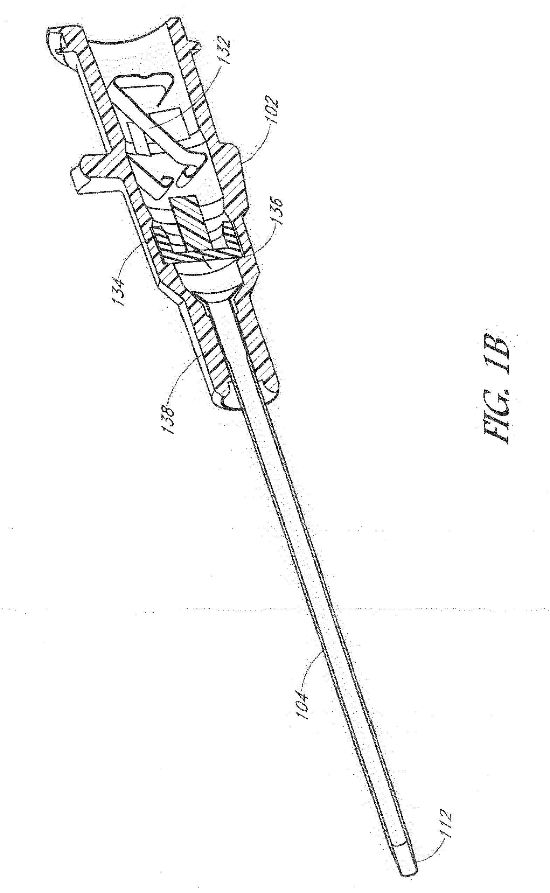

With reference now FIGs. 1A and 1B, components of a valved catheter assembly 100 with a blood collection holder 106 are shown. The IV catheter assembly 100, which may more broadly be referred to as a needle assembly or a needle device, is shown comprising a catheter tube 104, a catheter hub 102, a needle 108, a rubber shield 252, and a blood collection holder 106. The catheter hub 102 can have a female Luer at a proximal end for receiving a male Luer in a Luer fit, optionally with external threads.

The catheter tube 104 can be fixed to the catheter hub 102, such as with a bushing or a ferrule. Additional description with respect to the catheter hub 102 and the catheter hub 104 are further described below with respect to FIG. 1B.

A needle 108, which has a change in profile 144 proximal of a distal needle tip 110, can be inserted through the proximal opening of the catheter hub 102 with the distal needle tip 110 protruding from the distal opening 112 of the catheter tube 104 in a ready to use position. The needle 108 can also have a proximal needle tip 200 at an opposed end of the needle 108 from the distal needle tip 110. The change in profile 144 can be a crimp, a material buildup, or a sleeve, is configured to engage the needle guard and prevent the needle guard from displacing distally off of the needle. In some examples, the change in profile can be omitted and the needle guard can cover the needle tip without using the change in profile.

The needle guard 132 (FIG. 1B), which is understood to include structural features for guarding the needle tip from unintended needle sticks, is configured to be removed with the needle 108 following successful venipuncture while the valve 136 and valve actuator 134 are configured to remain with the catheter hub 102 for controlling fluid flow therethrough. The actuator 134 is configured to be pushed distally by a male tip of a medical implement, such as a syringe or an IV connector, into the valve 136 to open the valve for fluid flow. Further information regarding the needle guard 132 is discussed in U.S. Patent No. 8,568,372. In alternative embodiments, the needle guard 132 can embody multiple components that cooperate to block the needle tip from unintended needle sticks. For example, the needle guard can comprise a spring loaded needle carrier having a needle attached thereto. Following successful venipuncture, a release tab can be pressed to release the spring to then move the needle carrier and needle inside a protective barrel to block the needle tip from unintended needle sticks. Further information regarding various aspects of valved catheter assemblies and components thereof are discussed in PCT patent applications PCT/EP2016/069619 and PCT/EP2016/069643 and U.S. Patent No. 9,114,231.

A cannula hub or needle hub 106a can be incorporated at a distal end of the blood collection holder 106 to receive the needle 108, which can have a needle tip distal of the needle hub and a second needle tip located inside the interior of the needle holder for puncturing a septum on a vacutainer. Thus, when assembled with the catheter hub 102, the present assembly comprises a catheter hub having a catheter tube and a needle with two sharpened needle tips at two opposing ends of the needle shaft. For example, a catheter hub can be provided with a catheter tube, a needle can project through the catheter tube and has a first sharpened needle tip extending out a distal opening of the catheter tube. The needle can have a proximal section extending proximally of the proximal opening of the catheter hub and wherein the proximal section of the needle has a second sharpened needle tip.

In the present embodiment, the needle hub 106a is integrally formed with the body of the blood collection holder 106. For example, the needle hub can be molded with the body of the holder. Additional detail regarding the blood collection holder 106 and an associated rubber sleeve 252, also known as a multi-sample Luer adapter or MSLA, are described below with respect to FIGs. 2A-3C. In addition to embodying structures for securing the needle to a nose section of the needle hub, the blood collection holder 106 of the present embodiment can be sized and shaped to accept a vacutainer in its interior. For example and as further discussed below, the blood collection holder 106 can incorporate a body section having an interior sized and shaped to receive a vacutainer.

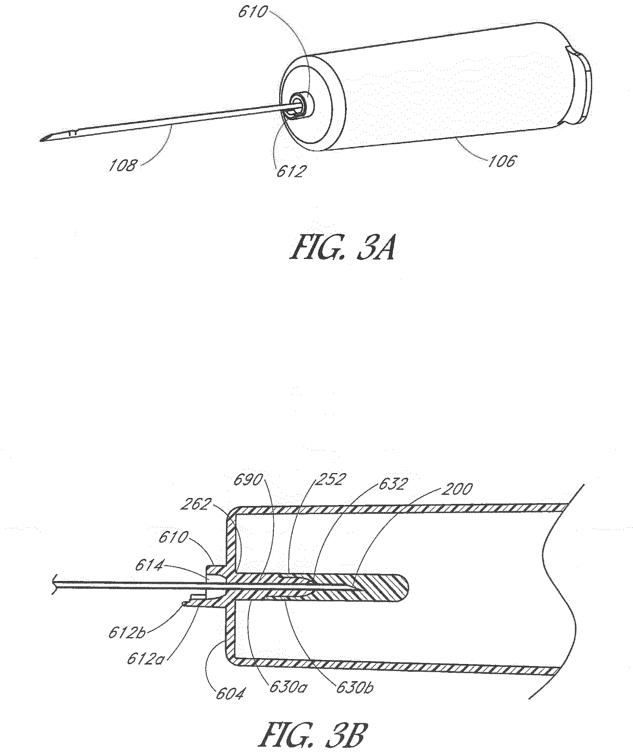

A rubber shield, deformable seal or self-sealing needle sheath 252, otherwise known as MSLA, can be attached to the interior of the blood collection holder 106 over a second end of the needle (FIG. 3B). In a ready to use position before insertion of a vacutainer into the blood collection holder 106, the rubber shield 252 can axially surround the proximal end of the needle 108 and cover the proximal needle tip 200 of the needle 108. The rubber shield 252 (FIG. 1A) can have a main body 254, a proximal end 256, and a distal end 258. In embodiments, the main body 254 can be generally cylindrical. The self-sealing needle sheath 252 can be made from a rubber or polymer material to allow for deformation in an axial direction of the needle 108. The open distal end 258 of the self-sealing needle sheath 252 can be sized and shaped to snap fit over a base in the interior of the holder and be assembled therewith for use.

The main body 254 and the distal end 258 of the rubber shield 252 can define an open interior space 260 and an open end that opens into the open interior space. The main body 254 can be defined by a sidewall having a thickness defined between its interior sidewall surface and its exterior sidewall surface. The distal end 258 can have a flange 262 extending radially outward from the main body. The distal end 258 having the flange 262 can have a radial thickness larger than the radial thickness of the main body 254.

Alternatively, the main body 254 can be formed in a different geometric shape, such as but not limited to a cone, a truncated cone, a pyramid, a truncated pyramid, a prism, a square, or a rectangle. In the case of the different geometric shape, the orientation of the geometric shape can be such that the main body can elastically deform in an axial direction of the needle 108.

The proximal end 256 of the rubber shield 252 can include a covering over the interior 260 of the rubber shield 252. The proximal end 256 can be considered as a sealed end of the rubber shield 252. The proximal end 256 can have a domed surface 264 to seal the proximal end of the rubber shield 252. Alternative shapes, such as a flat surface or conical surface can also be used. A hole or slit may be pre-formed in the surface 264 of the proximal end 256 for the needle 108 to pass through when the rubber shield 252 is elastically deformed in the axial direction. If a hole is pre-formed, the hole may be sized such that the surface tension of the blood from the patient prevents leakage or flow past the rubber shield 252 when the rubber shield is in a ready to use position without the needle 108 extending through the surface 264 of the proximal end 256. Alternatively, a slit, or multiple slits, can be pre-formed in the surface 264, such that the slit defines two flaps in the surface 264. When the needle 108 is inserted through the surface 264 of the proximal end 256, the needle can be inserted in the slit and separate the flaps from one another. When the needle 108 is not extending through the surface 264 of the proximal end 256, then the flaps can seal against one another.

As further described in FIGs. 2-4B below, the blood collection holder 106 can carry the cannula hub 106a at a distal end thereof. The blood collection holder 106 can have a main body 600 that is generally cylindrical. The blood collection holder 106 can have a distal wall 604 at one end of the main body 600 and a sidewall 607 defining the interior space 620. In some embodiments, the distal wall or end wall 604 can join with the main body 600 with a radius or fillet 606. From the distal wall 604, a mating flange 610 can extend in the distal axial direction. The mating flange 610 can be sized and shaped to project into the interior of the catheter hub 102 when assembled in the ready to use position, similar to a nose end of a needle hub of a standard catheter assembly. The mating flange 610 can embody a cylindrical projection. In some instances, the mating flange 610 and the interior support column 630 can be molded as an independent component, detachable from the end wall. For example, the combination mating flange 610 and interior support column 630 can be screwed on, push off, etc. from the end wall 604. Optionally, before use or after use, the combination mating flange 610 and interior support column 630 can be separately from the main body 600.

The mating flange 610 can have a mating flange opening 614 defined by an interior surface of the cylindrical projection. In some embodiments, the mating flange 610 can be a different geometric shape keyed to a corresponding interior shape of the catheter hub 102 in order to prevent rotation of the blood collection holder 106 relative to the catheter hub 102. In some embodiments, the cylindrical projection of the mating flange 610 can further include a mating projection or distal projection 612 extending farther in the distal direction from the mating flange 610. The mating projection 612 can have the same thickness as the thickness of the sidewall of the mating flange 610. In embodiments, the mating projection 612 can have a surface configured to push the needle guard 132 into engagement with the interior engagement projection formed with the catheter hub when the blood collection holder 106 is mated with the catheter hub 102. This allows the needle guard to be held in place in the ready to use position and during retraction of the needle following successful venipuncture, to prevent early activation.

At the opposite end, such as the proximal end of the main body 600, there can be an opening for receiving a vacutainer into the interior space 620 of the blood collection holder 106. Near or at the proximal end of the main body, a handle, flange, or gripping pad 602 (FIG. 1A) can project radially outward from the main body 600. The handle or flange 602 can be substantially rectangular in shape. In some embodiments, the blood collection holder 106 can have one handle 602 extending radially outward from the main body 600. In other embodiments, the blood collection holder 106 can have two or more handles 602 extending radially outward from the main body 600. With two or more handles 602, the handles can be spaced equidistantly around the main body 600 or spaced to be ergonomically comfortable for one handed gripping of the blood collection holder 106.

FIG. 1B shows a cross-sectional view of the catheter hub 102 taken along the axial direction with a catheter tube 104 and a bushing 138. The bushing 138 can be configured to wedge the proximal end of the catheter tube 104 against the interior wall surfaces of the catheter hub 102 to retain the catheter tube 104 to the catheter hub 102. Interiorly of the catheter hub 102, a septum or valve 136, an actuator or valve opener 134 and a needle guard 132, such as a safety shield or tip protector, are provided. The catheter hub 102 can be made from a single hub body as shown or from two or more hub bodies assembled together.

The septum or valve 136 can include at least one slit, defining elastically deformable flaps of the valve. The needle 108 can be inserted through the at least one slit such that the elastic flaps can be expanded by the hollow needle and closed when the needle is removed. The valve can be sized and shaped for multiple use by cooperating with the valve opener to open the one or more slits of the valve for fluid flow and closing upon removal of a male Luer tip that was placed into the catheter hub to push the valve opener in the distal direction. In some examples, the flaps can remain open and engaged with a valve opener 134 in a one-time activation configuration. The number of slits defining the number of flaps can vary, ranging from one slit to four or more slits defining two or more flaps, such as three or more flaps.

The catheter hub 102 can have a proximal end with an opening. The proximal end can also be provided with a female Luer fitting with exterior threads 103, forming a female threaded Luer. In some examples, the threads can be omitted and the proximal opening can function as a female Luer slip.

FIG. 1A shows the catheter hub 102 with a pair of wings. Embodiments of the catheter hub can also include embodiments without wings.

FIG. 2 illustrates a cross sectional view of the embodiment of the blood collection holder taken along line A-A of FIG. 1A. In the interior space 620 of the blood collection holder 106, an interior support column 630 extends proximally into the interior space 620 from the distal wall 604. The interior support column 630 can be generally cylindrical or conical in shape, extending from the axial center of the blood collection holder 106. The interior support column 630 can include a distal section 630a and a proximal section 630b. The distal section 630a, which is closer to the distal wall or end wall 604, can have a first outer diameter that is a larger diameter than a second outer diameter of the proximal section 630b. The transition of the outer diameter from the distal section 630a to the proximal section 630b can be stepped or tapered. The first outer diameter of the distal section 630a can be sized to correspond to the interior diameter of the rubber shield 252 for fixing the rubber shield 252 to the distal section 630a. As the rubber shield 252 can be elastically deformable and stretchable, the first outer diameter can be larger than the interior diameter of the rubber shield 252 when the rubber shield is in its relaxed state. Accordingly, the rubber shield 252 can be fitted over the distal section 630a to fix the rubber shield 252 to the distal section 630a. In some embodiments, the proximal section 630b can have a second outer diameter than the smaller interior diameter of the rubber shield 252. The smaller second outer diameter of the proximal section 630b can allow for space to accommodate the elastic deformation of the rubber shield 252 when the rubber shield 252 compresses in the axial direction towards the distal wall 604.

A bore 632 can extend through the interior support column 630 and through to the mating flange opening 614. The bore 632 can be sized and shaped to accommodate the needle 108. The bore 632 can be sized for an interference press fit with the needle 108 and/or can include a gap, or glue well, sufficient for adhesive to fix the needle 108 to the bore 632. The bore 632 extends through the needle hub 106a and the interior support column 630.

FIGs. 3A and 3B illustrate the needle 108 and the blood collection holder 106 in an attached configuration. FIG. 3A shows a perspective view of the needle 108 and the blood collection holder 106 in an attached configuration. FIG. 3B shows a cross sectional view of the embodiment of FIG. 3A taken across the axis of the needle assembly. The blood collection holder 106 can be mated with the needle 108 and the rubber shield 252. The rubber shield 252 can mate with the interior support column 630, extending proximally into the interior space 620 from the distal wall or end wall 604. The interior support column 630 can include a distal section 630a and a proximal section 630b. The distal section 630a of the interior support column 630 that is closer to the distal wall 604 can have a first outer diameter that is a larger diameter than a second outer diameter of the proximal section 630b.

In some embodiments, the rubber shield 252 can be elastically deformable and stretchable, such that the first outer diameter of the distal section 630a can be larger than the interior diameter of the rubber shield 252 when the rubber shield is in its relaxed state. Accordingly, the rubber shield 252 can be fitted over the distal section 630a to fix the rubber shield 252 to the distal section 630a. In other embodiments, the rubber shield 252 can have a rigid structure near its distal end 258 and have an interference fit with the first outer diameter of the distal section 630a.

The rubber shield 252 can be fitted such that the flange 262 of the rubber shield 252 abuts the distal wall 604. Additionally, the rubber shield 252 can be secured to the distal section 630a by way of a mechanical means such as a clip, barbs, detents, or by adhesive. In some embodiments, the rubber shield 252 can have a relaxed fitment over the distal section 630a and be secured by the clip, barbs, detents or adhesive. This may facilitate easier assembly.

The transition of the outer diameter from the distal section 630a to the proximal section 630b can be stepped or tapered. Proximal to the distal section 630a, the proximal section 630b of the interior support column can have a smaller second outer diameter than the first outer diameter of the distal section 630a. The smaller second outer diameter of the proximal section 630b can allow for space to accommodate the elastic deformation of the rubber shield 252 when the rubber shield 252 compresses in the axial direction towards the distal wall 604 due to insertion of a vacutainer into the blood collection holder 106.

The proximal needle tip 200 can be located inside the interior of the rubber shield 252. As such, the proximal needle tip 200 is located at an intermediary position between the interior support column 630 and the proximal end 256 of the rubber shield 252. This way, the rubber shield 252 surrounds the proximal needle tip 200, separating the proximal needle tip 200 from the remainder of the interior space 620 of the blood collection holder 106.

In this way, in the ready to use position, the rubber shield 252 can act as a sanitary shield to prevent contamination of the proximal needle tip 200 and to seal the needle from blood flashback during replacement of the vacutainer with a new or different vacutainer. When a vacutainer is inserted into the interior space 620 of the blood collection holder, the vacutainer, such as the septum on the vacutainer, can compress and elastically deform the proximal end 256 of the rubber shield 252 axially towards the distal wall 604. The proximal end 256 of the rubber shield 252 can be elastically deformed past the proximal needle tip 200, such that the proximal needle tip 200 extends proximally of the proximal end 256 of the rubber shield 252 and into the vacutainer, such as through the septum of the vacutainer.

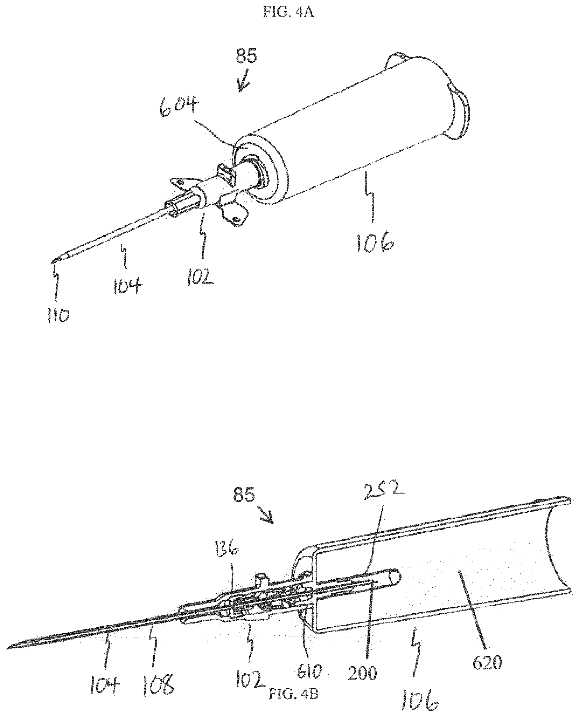

At the distal end of the bore 632, the mating flange 610 extends distally of the distal wall 604 of the blood collection holder. The cylindrical projection of the mating flange 610 can further include a mating projection or distal projection 612 extending farther in the distal direction from the mating flange 610. The mating projection 612 can include a primary proximal portion 612a, an arcuate section extending farther in the distal direction from the cylindrical projection that is the mating flange 610. The mating projection 612 can have a distal key portion 612b extending distally from the primary proximal portion 612a. The distal key portion 612b can have a similar outer arcuate shape as the primary proximal portion 612a and the mating flange 610. The distal key portion 612b can have a radially inner flat portion to set the needle guard 132 in the catheter hub, as described below with respect to FIG. 4B.

FIGs. 4A and 4B illustrate the catheter hub 102, the needle 108, and the blood collection holder 106 in an attached or assembled configuration, which can be referred to as a blood collectable peripheral intravenous catheter assembly 85, or a catheter and holder assembly 85. FIG. 4A shows a perspective view of the catheter hub 102, the needle 108, and the blood collection holder 106 in the attached or assembled configuration. In the assembled, ready to use position of the attached configuration, the catheter hub 102 abuts the distally facing surface of the distal wall 604 of the blood collection holder 106. The needle 108 extends through the catheter hub 102 and the catheter tube 104, with a needle tip 110 extending distally of the catheter tube opening.

FIG. 4B shows a cross sectional view of the embodiment of FIG. 4A taken along plane extending lengthwise of the of the needle assembly. FIG. 4B shows the cross sectional view of the needle assembly in an assembled, ready to use position. In the ready to use position, the catheter hub 102 abuts the distally facing surface of the distal wall 604 of the blood collection holder 106 with the mating projection 612 and the mating flange 610 of the blood collection holder 106 projecting into the interior of the catheter hub 102.

The needle 108 extends through the catheter hub 102 and the catheter tube 104, thereby extending through the valve 136 and the valve opener 134, which are located in the interior of the catheter hub 102. As the needle 108 provides a through connection, a flow lumen, from the distal needle tip 110 to the proximal needle tip 200, the needle 108 provides a through connection from the distal needle tip 110 to the space between the proximal needle tip 200 and the rubber shield 252 in the interior space 620 of the blood collection holder.

In the ready to use position, the needle guard 132 is positioned along the needle 108. For example, the needle can project through an opening on a proximal wall of the needle guard. Upon retraction of the needle, the crimp or change in profile 144 formed with the needle 108 can move proximally of two distal walls at an end of two arms of the needle guard. As the needle moves further proximally, the crimp or change in profile can engage the opening on the proximal wall to retain the needle guard 132 on the needle and not fall off distally of the needle tip.

The needle guard 132, which is understood to include structural features for guarding the needle tip from unintended needle sticks, is configured to be removed with the needle 108 following successful venipuncture. The valve 136 and valve actuator 134 are configured to remain with the catheter hub 102, such as inside the interior of the catheter hub, for controlling fluid flow therethrough.

In an exemplary embodiment, as further described in the reference of U.S. Patent No. 8,568,372, the change in profile 144 of the needle 108 can be configured to engage with an outer circumference defining a proximal opening on the rear wall or proximal wall of the needle guard 132 so that the needle guard 132 can be removed from the catheter hub 102 with the needle 108. The proximal wall of the needle guard 132 is slidably located between the location of the change in profile 144 and the proximal end of the needle 108. Thereby, the proximal wall of the needle guard 132 can be prevented from passing the change in profile 144 and from becoming separated from the needle 108.

The fitment of the needle guard 132 in the catheter hub 102 can also serve to prevent rotation of the needle guard 132. The needle guard 132 can be fitted in the catheter hub 102 such that rotation of the needle guard 132 relative to the catheter hub 102 can be prevented or minimized.

The radially inner flat portion of the distal key portion 612b of the blood collection holder 106 can contact a portion of the needle guard 132. In an exemplary embodiment, the needle guard 132 can have a corresponding proximal flat surface or edge for contact with the flat portion of the distal key portion 612b.

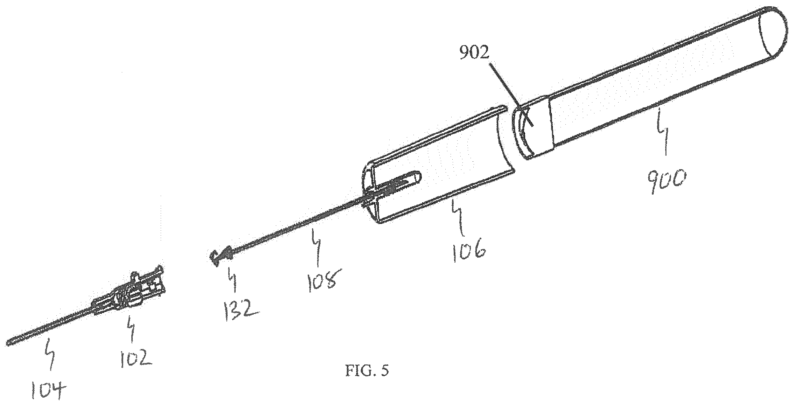

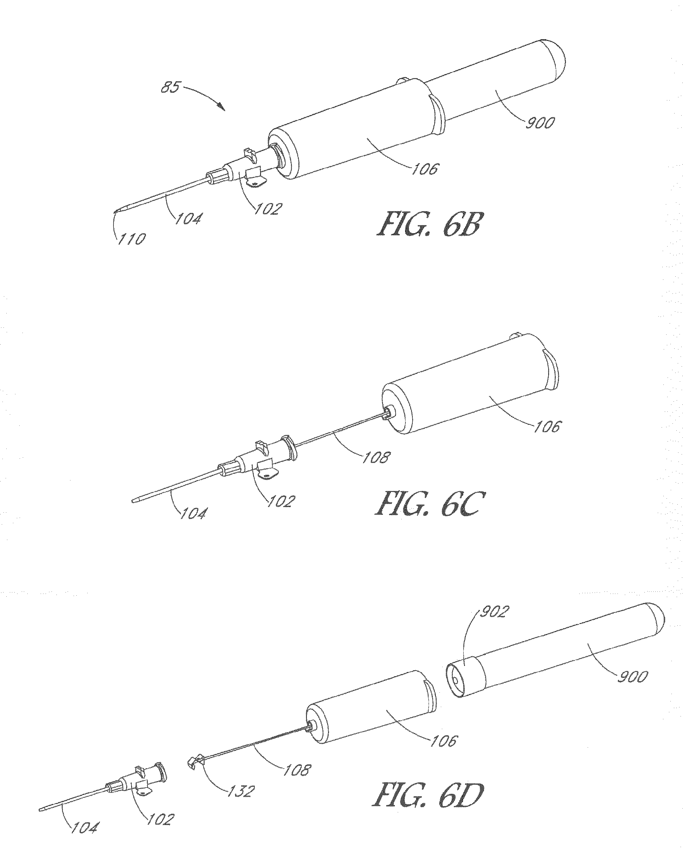

FIG. 5 illustrates an exploded cross-sectional view of an exemplary embodiment of the catheter assembly 100 and a vacutainer 900 with a septum 902. The catheter hub being spaced from the needle and the needle guard covering the needle tip resembles a configuration in which activation and separation occur following successful venipuncture. Further description regarding the operation of the catheter assembly 100 and the actuated position when the needle 108 is withdrawn from the catheter hub 102 can be found below with respect to FIGs. 6D and 7C.

In the actuated position following initial placement of the catheter into the vein for blood sampling then subsequently advanced for peripheral venous access, the blood collection holder 106 is separated from the catheter hub 102. The needle is withdrawn proximally from the catheter tube 104 and the catheter hub 102 following peripheral venous access and following the blood sampling. When the needle 108 is withdrawn from the catheter hub 102, the needle guard 132 stays coupled to the needle 108 and is withdrawn from the catheter hub 102 with the needle. The needle guard 132 can cover the distal needle tip 110 to prevent accidental needle stick when the needle 108 is withdrawn from the catheter hub 102 and the crimp or material build up on the needle near the distal needle tip prevents the needle guard from displacing distally off of the needle.

At least two processes or methods of usage of the catheter and holder assembly 85 can be envisioned (not being according to the claimed invention). FIGs. 6A-6D illustrate a first process for usage wherein blood sampling is performed while the blood collection holder 106 is mated, and in contact, to the catheter hub 102. FIGs. 7A-7C illustrate a second process for usage wherein the catheter tube 104 is advanced into a patient's vein, thereby separating the blood collection holder 106, spaced the holder, from the catheter hub 102, before performing blood sampling.

With reference initially to FIGs. 6A-6D, the first process for usage of the catheter and holder assembly 85 is shown, illustrating different stages of the assembly as configured for blood sampling and for catheterization. In the configuration illustrated, blood sampling can be performed with the blood collection holder 106 mated to the catheter hub 102, such that the distal needle tip 110 of the needle extends distally of the distal opening of the catheter tube 104 during blood sampling.

FIG. 6A illustrates the catheter and holder assembly 85 in a ready to use position with the blood collection holder 106, which has a housing with a proximal opening sized and shaped to receive a vacutainer, mated to the catheter hub 102. In the ready to use position, as described with respect to FIGs. 1-5, the distal needle tip 110 extends distally of a distal opening of the catheter tube 104 such that the distal needle tip 110 can be used to access the vein like a conventional blood collection procedure. Upon successful accessing the vein, the distal needle tip 110 and the distal end of the catheter tube 104 are located inside the vein, thereby allowing needle 108 to provide a fluid communication between the vein and the interior space 620 of the blood collection holder 106, and particularly to a proximal needle section of the needle having a second sharpened needle tip 200 that is covered by a rubber sheath or shield 252 to limit or prevent blood flashback into the interior space of the holder 106 (FIG. 4B).

The medical professional can then hold the catheter assembly 100 with one hand in a steady manner to avoid movement of the catheter assembly 100 relative to the vein to enable blood collection. In some embodiments, the catheter hub 102 can be provided with a pair of wings 120 to facilitate securement of the catheter hub 102 to a patient following venipuncture. The pair of wings can be flanges extending radially outward from the catheter hub 102 and flexible to contact the patient's skin.

FIG. 6B illustrates blood sampling by insertion of a vacutainer 900 into the interior space 620 of the blood collection holder 106. The vacutainer 900 is inserted while the blood collection holder 106, such as the distal end of the holder, is still in contact with the catheter hub 102. As described with respect to FIG. 3B, the insertion of the vacutainer 900 can axially deform the rubber shield 252 in the interior space 620 of the blood collection holder 106 (FIG. 4B) such that the rubber shield 252 is deformed distally past the proximal needle tip 200 by the septum 902 on the vacutainer 900. As the vacutainer 900 is fully inserted, the proximal needle tip 200 can pierce the septum 902 of the vacutainer and be inserted into the vacutainer 900, such that the interior of the vacutainer 900 can be in a fluid communication with the needle lumen.

With the fluid connection through the needle 108, blood sampling with the vacutainer 900 can thus be performed while the blood collection holder 106 stays mated to, and in contact with, the catheter hub 102.

Upon removal of the vacutainer 900 after successful blood sampling, the rubber shield 252 can return to its ready to use position, thereby covering the proximal needle tip 200 of the two-sharpened-tip-needle and preventing blood leakage through the proximal needle tip 200. The elastic deformation of the rubber shield 252 allows for repeated blood sampling by serially inserting additional vacutainers 900 into the interior space 630 of the holder 106 while also preventing blood leakage when each vacutainer is removed from the blood collection holder 106.

After the last of the vacutainers 900 is removed from the holder 106, the needle 108 and the holder 106 can retract proximally away from the catheter hub 102. FIG. 6C illustrates a post-blood sampling position when the medical professional advances the catheter tube into the vein for catheterization. After successful blood sampling has been completed, the medical professional can advance the catheter hub 102 and the catheter tube 104 to place the catheter tube into the vein as normally done per peripheral IV catheter (PIVC) procedures. In this way, the medical professional can maintain the position of the blood collection holder 106, and thereby the needle 108, relative to the vein, and advance the catheter tube into the vein by moving the catheter hub in the distal direction.

Upon advancing of the catheter hub 102 and the catheter tube 104, the medical professional can fully remove the blood collection holder 106 and the needle 108 attached to the holder from the catheter hub 102 by retracting the blood collection holder 106 in the proximal direction relative to the catheter hub 102.

FIG. 6D illustrates the catheter assembly 100 after successful catheterization and separation of the blood collection holder 106 from the catheter hub 102. Additionally filled vacutainers 900 from the prior blood sampling procedure are not shown. Optionally, the last of the vacutainers 900 can remain with the blood collection holder 106 while the blood collection holder 106 is retracted away from the catheter hub 102. Then the last of the vacutainer can be removed from the blood collection holder 106. In other words, the needle 108 and the holder 106 can be removed from the catheter tube and the catheter hub with or without the vacutainer 900 located in the holder.

On withdrawal of the hollow needle 108 from the catheter hub 102 following successful venipuncture, a change in profile 144 provided near the distal needle tip 110 and having the form of a radial projection on the hollow needle, such as by crimping, engages with the safety shield 132 so that the safety shield 132 can be removed from the catheter hub 102 with the needle 108. The safety shield 132 can then act as a shield or cover the distal needle tip 110 of the needle 108 as it is withdrawn from the catheter hub 102.

In an exemplary embodiment, the safety shield 132 can have two arms that can move to cover the distal needle tip 110. As the distal needle tip 110 moves proximally of distal walls of the two arms, the two arms are no longer biased by the needle and can move, such as spring or deflect radially to disengage from the interior of the catheter hub 102. As the arms of the safety shield 132 move radially, the arms, or the distal walls of the arms, can cover the needle tip 110 to prevent unintended needle sticks. That is, the distal walls can move from a position to the side of the needle tip to a position distal of the needle tip to prevent unintended needle sticks. In some examples, the needle shield 132 can have only one arm and one distal wall at an end of the one arm. In other examples, the change in profile can include a sleeve, a notch, or a material buildup on the shaft of the needle. Using a notched needle near the distal tip allows blood flashback to be viewed in the annular space between the needle and the catheter tube. Further information regarding the safety shield 132 is discussed in U.S. Pat. No. 7,736,339.

Following removal of the needle 108 after successful catheterization, the one or more flaps of the valve 136 located inside the catheter hub 102, due to their elastic properties, close the one or more slits through the depth of the valve disk or disc so that no blood or substantially no blood can flow from the catheter tube 104 into the proximal chamber of the catheter hub 102, and out through the proximal opening of the catheter hub 102. In this way, the valve 136 prevents further blood spill or flow.

Accordingly, the process described in FIGs. 6A-6D can provide for both blood sampling and catheterization for IV infusion with only one needle stick using the catheter and holder assembly 85 of the present invention. In comparison, prior art procedures can involve a first needle stick using a blood collection needle to collect blood and then after the blood collection and the blood collection needle is removed, the procedures can involve a second needle stick for PIVC access for IV infusion. In the present embodiment, the blood collection holder 106, as previously described, has an integrated needle hub 106a (FIG. 1A) for securing a needle 108 having two sharpened needle tips, a distal needle tip 110 and a proximal needle tip 200. The blood collection holder 106 further comprises a housing or main body 600 attached to the needle hub 106a, or said differently - the needle hub 106a is attached to a main body of a blood collection holder 106. The housing being generally cylindrical and having an interior that is sized and shaped to receive a vacutainer. The proximal needle tip 200 can be positioned in the interior of the housing 600 for puncturing a septum 902 on the vacutainer to provide fluidic communication between the distal needle tip 110, the needle lumen, the proximal needle tip 200, and the interior of the vacutainer 900, when the needle is not shielded by the rubber sheath or shield.

FIGs. 7A-7D illustrate a second process for usage of the catheter and holder assembly 85 wherein blood sampling is performed after the medical professional has advanced the catheter tube 108 into the patient, similar to normal PIVC access, but before fully removing the needle from the catheter tube lumen. Thus, before the needle 108 that is attached to the blood collection holder 106 is completely removed from the catheter tube, blood collection can be performed after the blood collection holder 106 has separated from the catheter hub 102.

FIG. 7A illustrates the catheter and holder assembly 85 in a ready to use position with the blood collection holder 106 mated to the catheter hub 102. In the ready to use position, as described with respect to FIGs. 1-5, the distal needle tip 110 extends distally of a distal opening 112 of the catheter tube 104 such that the distal needle tip 110 can be used to access the vein. The distal needle tip 110 is then retracted proximally of the distal opening 112 of the catheter tube 104 as the catheter tube is further advanced into the vein. At this time, primary and secondary flashbacks can be confirmed. While the catheter tube is further located inside the vein and the needle is retracted in the proximal direction but not completely out of the lumen of the catheter tube 104, the needle 108 maintains fluid communication between the vein and the interior space 620 of the blood collection holder 106, such as through to the proximal needle tip 200.

The medical professional can then either partially or fully advance the catheter tube 104 into the patient's vein prior to blood sampling. Optionally, for the procedure of FIGs .6A-6D or 7A-7D, a tourniquet may be used with the present catheter and holder assembly 85 as conventionally practiced. Where not permitted, such as under restrictions by certain local rules, a tourniquet may not be used and if used where not permitted, the first bottle or vacutainer may need to be discarded.

The medical professional can then hold the catheter assembly 100 with one hand sufficiently steady to prevent movement of the catheter assembly 100 relative to the vein. In some embodiments, the catheter hub 102 can be provided with a pair of wings 120 to facilitate securement of the catheter hub 102 to a patient following venipuncture. The pair of wings can be flanges extending radially outward from the catheter hub 102 and flexible to contact the patient's skin.

FIG. 7B illustrates a ready to sample position of the catheter and holder assembly after either partial or normal full advancement of the catheter tube 104 into the vein. With the advancement of the catheter hub 102 to advance the catheter tube 104 and proximal retraction of the needle during the catheter tube advancement, the blood collection holder 106 and the catheter hub 102 can be separated, or physically spaced, along the axial direction of the needle while still coupled by the needle 108 and the lumen of the catheter tube. In the ready to sample position, the needle 108, which is fixed to the blood collection holder 106, can still be in the vein of the patient or can be recessed from the distal opening of the catheter tube and located in the lumen of the catheter tube such that the needle, which is in fluid communication with the lumen of the catheter tube, provides a fluid connection between the vein and the proximal needle tip 200.

In practice, the advancement of the catheter hub 102 with the distal needle tip 110 recessed or retracted in the proximal direction from the distal opening of the catheter tube can serve as a safeguard against accidental advancement of the needle 108 in the patient.

FIG. 7C illustrates blood sampling by insertion of a vacutainer 900 into the interior space 620 of the blood collection holder 106 so that the proximal needle tip 200 punctures the septum on the vacutainer to open fluid communication with the interior of the vacutainer. The vacutainer 900 is inserted into the blood collection holder 106 after the blood collection holder 106 retracts from the catheter hub 102 but wherein the needle is still in fluid communication with the catheter tube. As described with respect to FIG. 3B, the insertion of the vacutainer 900 can axially deform the rubber shield 252 in the interior space 620 of the blood collection holder 106 such that the rubber shield 252 is deformed distally past the proximal needle tip 200 to expose the needle tip 200. As the vacutainer 900 is fully inserted, the proximal needle tip 200 can pierce the septum and be inserted into the vacutainer 900, such that the vacutainer 900 can be in a fluid connection with the proximal needle tip 200.

With fluid connection between the vacutainer 900 and the needle 108, blood sampling can thus be performed while the blood collection holder 106 stays mated to the catheter hub 102 via the needle 108.

Upon removal of the vacutainer 900 after successful blood sampling, the rubber shield 252 can return to its ready to use position, thereby covering the proximal needle tip 200 and preventing blood leakage through the needle and out the proximal opening of the catheter hub, as shown and described above with reference to FIG. 3B. The elastic deformation of the rubber shield 252 allows for repeated blood sampling with a plurality of vacutainers, while also preventing blood leakage when each of the vacutainers is removed from the blood collection holder 106.

After successful blood sampling has been completed, the medical professional can then remove the blood collection holder 106 and the attached needle 108 from the catheter hub 102. The medical professional can also fully remove the blood collection holder 106 and the needle 108 from the catheter hub 102 by retracting the blood collection holder 106 axially in the proximal direction relative to the catheter hub 102. The needle 108 and the holder 106 can be removed from the catheter tube and the catheter hub with or without the vacutainer 900 located in the holder.

FIG. 7D illustrates the catheter assembly 100 after successful catheterization and separation of the blood collection holder 106 from the catheter hub 102, including complete removal of the needle from the catheter tube and the catheter hub. There may be additional filled vacutainers that are not shown.

On withdrawal of the hollow needle 108 from the catheter hub 102 following successful venipuncture, a change in profile 144 provided near the distal needle tip 110 and having the form of a radial projection on the hollow needle, such as by crimping, engages with the safety shield 132 so that the safety shield 132 can be removed from the catheter hub 102 with the needle 108, as previously described elsewhere herein. The safety shield 132 can then act as a shield or cover the distal needle tip 110 of the needle 108 as it is withdrawn from the catheter hub 102.

Following removal of the needle 108 after successful catheterization, the one or more flaps of the valve 136 (FIGs. 1B and 4B), due to their elastic properties, close the one or more slits through the depth of the valve disk or disc so that no blood or substantially no blood can flow out through the proximal opening of the catheter hub 102. In this way, the catheter hub 102 prevents further blood loss or flow.

Accordingly, using a catheter and holder assembly 85 of the present invention, the process described in FIGs. 7A-7D can provide for blood sampling and catheterization with only one needle stick. In contrast, prior art devices require a first needle stick with a blood sampling device to obtain blood samples via vacutainers. The blood sampling device is then removed from the patient after the desired number of samples, such as tubes, have been taken. A second needle stick with a catheter assembly is then carried out to obtain peripheral IV access. After successful venipuncture, the catheter hub can then connect with a drip line. However, the catheter and holder 85 in accordance with aspects of the present invention can perform both the blood sampling function and the peripheral IV access with only a single needle stick. The present assembly thus reduces waste, by utilizing fewer components and at least one less needle, and reduces the number of needle sticks to one, which can minimize needle stick injuries and potential stress on the patient from the possibly receiving more than one needle sticks.

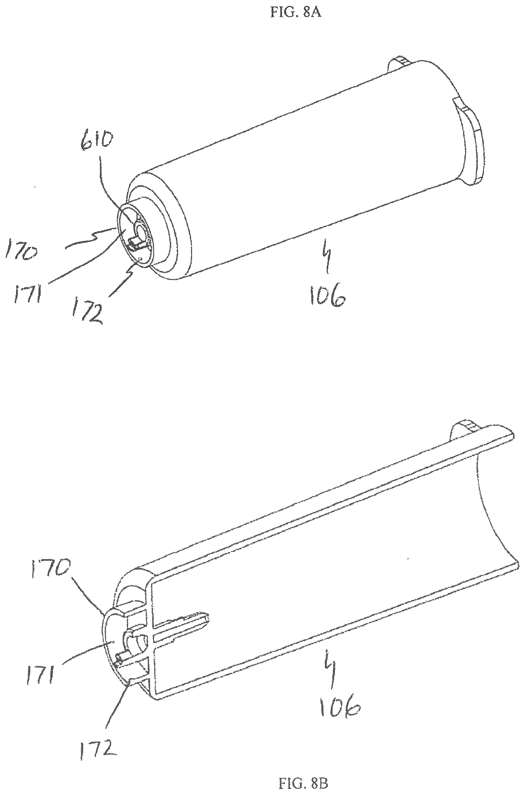

FIGs. 8A and 8B illustrate an alternative embodiment of the blood collection holder 106. The alternative embodiment provides for a protrusion cover 170 around the mating flange 610, with a gap or space between the two. As previously described, the mating flange 610 is configured project into the catheter hub. A stopper projection 172 can be provided on the inside surface of the protrusion cover 170, which can also be called a distal ring flange. The protrusion cover 170 is sized and shaped to project around the exterior of the catheter hub, at the proximal end of the catheter hub, and the mating flange 610 is configured to project into the proximal opening of the catheter hub. The protrusion cover can prevent accidental or early separation of the catheter hub 102 from the blood collection holder 106 during cannulation and blood sampling. Accidental separation of the catheter hub 102 and the blood collection holder 106 can be unnerving for a medical professional and creates a risk of inadvertent needle movement while the medical professional adjusts to the relative movement of the catheter hub 102.

In order to prevent accidental movement, the blood collection holder 106 can have a protrusion cover 170, which can be a cylindrical projection extending distally from the distal wall 604 of the blood collection holder 106. The cylindrical projection can have a wall structure that is spaced radially outward from the mating flange 610, such that the protrusion cover 170 and the mating flange 610 are concentric with each other, and the protrusion cover extends distally from the distal wall 604 to cover a proximal portion of the catheter hub 102 when mated with the blood collection holder 106. In an example, the exterior surface of the protrusion cover 170 can also be profiled, such as include detents, engagement features, interference surface, to engage a protective sheath that is placed over the catheter assembly during packaging and shipping, if the catheter assembly and blood collection holder were packaged as a single assembly.

On the interior surface 171 of the protrusion cover 170, a stopper projection 172 can extend radially inwardly from the interior surface 171. The stopper projection 172 can be a single raised lip or bump or an annular protrusion that is formed around the interior circumference of the protrusion cover 170. Optionally, the stopper projection 172 can be non-continuous and formed around the interior circumference by providing a plurality of spaced apart individual bumps. The stopper projection 172 is configured to contact a rear exterior feature or surface of the catheter hub, such as the exterior threads of the catheter hub, to restrict relative movement.

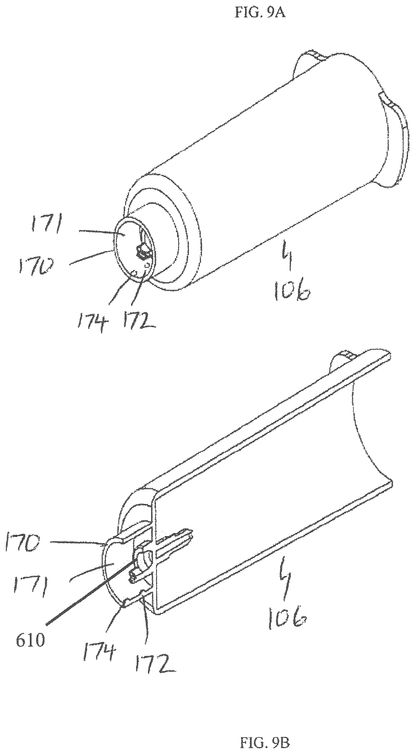

FIGs. 9A and 9B illustrate another alternative embodiment of the blood collection holder 106. The alternative embodiment provides for a protrusion cover 170 formed around the mating flange 610 and provided on the interior surface with two stopper projections 172, 174, having a space or gap in between. Further to the cover protrusion 170 and the stopper projection 172 described in FIGs. 8A and 8B, the cover protrusion 170 of the present embodiment can include a second stopper projection 174 located axially farther from the distal wall 604, or distally spaced from the distal wall, of the blood collection holder 106 and spaced from the first stopper projection 172. Like the first stopper projection 172, the second stopper projection 174 can be a single raised lip or bump or an annular protrusion that is formed around the interior circumference of the protrusion cover 170. Optionally, the second stopper projection 174 can be non-continuous and formed around the interior circumference by providing a plurality of spaced apart individual bumps. The second stopper projection 174 can be aligned radially with the first stopper projection 172. Alternatively, the second stopper projection 174 can be offset radially along the circumference of the interior of the cover protrusion. As shown, the second stopper projection 174 is located distal of the first stopper projection 172.

With the second stopper projection 174, even if the catheter hub is accidentally moved past the first stopper projection 172, the catheter hub 102 can be retained by the second stopper projection 174. The second stopper projection 174 can be viewed as a backup to the first stopper projection 172. The present embodiment can be viewed as a blood collection holder 106 having two separation stops, or one stop with one backup stop.

The first stopper projection 172 and the second stopper projection 174 can be the same size and the same shape. Alternatively, the first stopper projection 172 and the second stopper projection 174 can be of different sizes, such that different levels of force can be applied to the catheter hub to require different level of forces to overcome the two stopper projections. For example, the second stopper projection 174 can require a higher separation force than the first stopper projection 172 by incorporating a more inwardly surface structure or lip from the interior surface 171 of the protrusion cover 170 than the first stopper projection.

As understood from the embodiments of FIGs. 8A-9B, additional stopper projections, such as more than two rows of stopper projections, can be provided to provide additional blocks from separation of the catheter hub 102 from the blood collection holder 106. Additionally, alternative forms of stopper projections can be used. Although the exemplary embodiments use domed, annular lips, protrusions, or bump stopper projections, the stopper projections could alternatively be of another geometric shape, such as a rectangle, different shapes, detents, relatively sharp gripping structures like barbs, etc., or be an arcuate flange on the interior of the cover protrusion. The stopper projections can be of different shapes from one another.

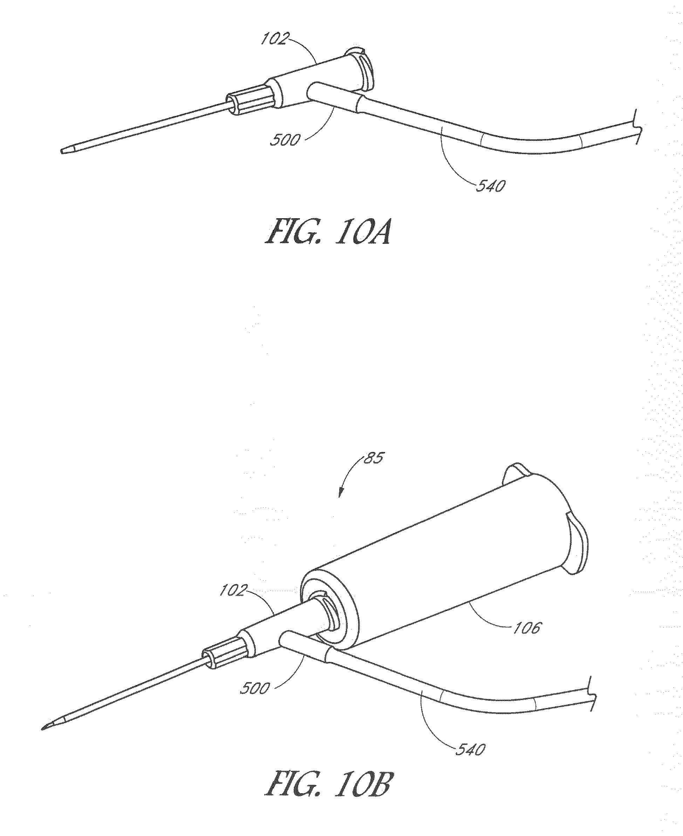

FIG. 10A and 10B illustrate an alternative embodiment of the catheter hub 102 wherein the body of catheter hub is provided with side fluid port and tubing, also known as an integrated catheter hub. FIG. 10A illustrates a perspective view of the alternative embodiment of the catheter hub 102 wherein the catheter hub 102 is an integrated catheter hub and FIG. 10B illustrated a perspective view of a catheter and holder assembly 85 having the catheter hub of FIG. 10A and a blood collection holder 106, which can be one of the holders discussed elsewhere herein.

The integrated catheter hub 102 can include a side fluid port 500 extending obliquely from the generally cylindrical body of the catheter hub 102. A tubing 540 can be connected to the side fluid port 500 at its first end and the opposite end of the tubing can be connected to a fluid adapter, such as a male or a female needleless valve, which a syringe without a needle can attach and infuse fluids, such as medication. The tubing 540 can have a lumen for fluid flow and provide a fluid connection between the catheter hub 102 through the side fluid port 500 and an external adapter. The external adapter can be any type of conventionally known adapter, such as a Luer connector or a conventional vent plug. Blood flow can be stopped by a seal or septum located at the proximal end of the catheter hub 102, proximal of where the side fluid port 500 opens into the catheter hub so as to not interfere with fluid flow through the side fluid port 500. IV fluid or other medicaments can flow into the catheter hub 102 via connection from the external fluid adapter through the tubing 540 and through the side fluid port 500.

Additionally, the catheter hub 102 of the present embodiment can have a pair of wings projecting from the body of the catheter hub to aid in securement to the patient, such as an adhesive dressing.

FIG. 10B illustrates a perspective view of the integrated catheter hub 102 mated with a blood collection holder 106, which can be one of the holders 106 discussed above.

Additionally, although FIG. 10B shows mating with the blood collection holder 106 without a cover protrusion or stopper projections, alternative blood collection holders can include a cover protrusion or stopper projections for use with the integrated catheter hub 102. The side fluid port 500 and the tubing 540 can be arranged such that they do not interfere with the fitment of the protrusion cover when mating the blood collection holder 106 and the integrated catheter hub 102.

The operating process for a catheter assembly 102 using an integrated catheter hub 102 can be the same as that shown with the exemplary straight catheter with respect to FIGs. 6A-7D.

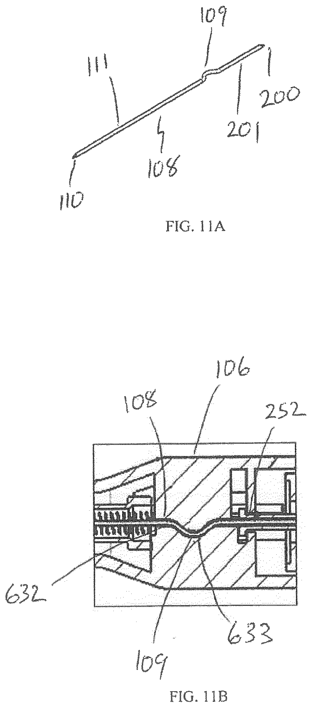

FIGs. 11A and 11B illustrate an alternative needle 108 wherein the shaft of the needle has a curve feature or curved section 109 for fitment with a corresponding shaped groove in the blood collection holder 106. The needle 108 can have the curve feature 109 at an intermediary position between the distal needle tip 110 and the proximal needle tip 200. The curve feature can be a simple C-shaped bend, such that the distal needle tip 110 and a distal section 111 of the needle shaft are axially aligned with the proximal needle tip 200 and a proximal section 201 of the needle shaft. Alternatively, the bend 109 can be a different shape, such as an S-shape, or a complex shape.

The bore 632 of the blood collection holder 106 can likewise have a corresponding slot or shape 633 for receiving or accommodating the curved section 109 of the needle 108 of FIG. 11A. The needle 108 can be fitted and snapped into the corresponding slot, or corresponding curved feature 633 of the blood collection holder 106. In some embodiments, the needle 108 can be forced into the corresponding slot 633 by axial insertion. In some embodiments, the blood collection holder, at least at the needle hub section of the holder, can be a two part construction with division along the bore, such that the needle 108 can be snapped into the corresponding slot 633 prior to assembly of the blood collection holder 106. The corresponding slot 633 can have a corresponding profile as the bend section 109 of the needle 108 and a slight undercut feature so that a slight force is needed to push the needle into the slot.

The combination of the corresponding slot 633 and the bend feature 633 can adequately secure the cannula mechanically, without needing additional adhesive. Alternatively, adhesive can be used or other bonding technique such as ultrasonic welding can be used to bond the needle 108 with the blood collection holder 106 even when using the bend feature 109 and the corresponding shaped slot 633.

As described above, embodiments of the components from the cited references can be implemented for the various components of the catheter assemblies of the present invention and discussed herein. For example, regarding the catheter hub, the body of the hub can be a wingless catheter hub, a winged catheter hub, a ported catheter hub, or a catheter adapter with an inner lumen with a valve or permanent septum.

Methods of making and of using catheter and holder assemblies and their components as described herein are not within the scope of the claimed invention. The methods are understood to include the use of a blood collection holder as a cannula hub with an IV catheter, both in straight IV catheter assemblies and integrated IV catheters, which have a side fluid port extending from the body of the catheter hub and has a tubing attached thereto, which then has a fluid adapter at the end of the tubing.

Although limited embodiments of catheter and holder assemblies, their components, and their applications for blood sampling and peripheral IV access have been specifically described and illustrated herein, many modifications and variations will be apparent to those skilled in the art. For example, the various features of a double tipped needle, rubber shield, and blood collection holder may incorporate alternate materials, etc. Furthermore, it is understood and contemplated that features specifically discussed for one blood collection holder embodiment may be adopted for inclusion with another blood collection holder embodiment, provided the functions are compatible. Accordingly, it is to be understood that the blood collection holder and its applications in catheter assemblies constructed according to principles of the disclosed devices, systems, and methods may be embodied other than as specifically described herein. The disclosure is also defined in the following claims. IV catheter assemblies and integrated IV catheters, which have a side fluid port extending from the body of the catheter hub and has a tubing attached thereto, which then has a fluid adapter at the end of the tubing.

Although limited embodiments of catheter and holder assemblies, their components, and their applications for blood sampling and peripheral IV access have been specifically described and illustrated herein, many modifications and variations will be apparent to those skilled in the art. For example, the various features of a double tipped needle, rubber shield, and blood collection holder may incorporate alternate materials, etc. Furthermore, it is understood and contemplated that features specifically discussed for one blood collection holder embodiment may be adopted for inclusion with another blood collection holder embodiment, provided the functions are compatible. Accordingly, it is to be understood that the blood collection holder and its applications in catheter assemblies constructed according to principles of the disclosed devices, systems, and methods may be embodied other than as specifically described herein. The disclosure is also defined in the following claims.

## Claims

1. A catheter and holder assembly (85) comprising:
a catheter hub (102) having a catheter tube (104) with a distal opening (112) attached to the catheter hub (102);
a needle (108) comprising a sharpened distal needle tip (110) which projects out the distal opening (112) of the catheter tube (104); and
a blood collection holder (106) having a housing (600) having an interior space (620) dimensioned to receive a vacutainer;
**characterized in that**
the blood collection holder (106) has a needle hub (106a) with a bore (632) having the needle (108) passing therethrough;
the needle (108) comprises a sharpened proximal needle tip (200); and
the catheter hub (102) is in contact with the blood collection holder (106) and the sharpened proximal needle tip (200) is located within the interior space (620) of the housing (600) in a ready to use position.

2. The catheter and holder assembly (85) according to claim 1, wherein the needle (108) comprises a notch, a crimp, a sleeve, or a buildup located proximally of the sharpened distal needle tip (110).

3. The catheter and holder assembly (85) according to claims 1 or 2, wherein the blood collection holder (106) comprises an end wall (604) and a sidewall (607) defining the interior space (620) and the needle hub (106a) is integrated with the end wall (604).

4. The catheter and holder assembly (85) according to any one of claims 1 to 3, wherein the sharpened proximal needle tip (200) is in the interior space (620) of the blood collection holder (106) when the catheter and holder assembly is in the ready to use position.

5. The catheter and holder assembly (85) according to any one of claims 1 to 4, wherein the blood collection holder (106) has a projection (612) that projects into the catheter hub (102) in the ready to use position.

6. The catheter and holder assembly (85) according to any one of claims 1 to 5, wherein a portion of the needle (108) that extends distally of the blood collection holder (106) is longer than a portion of the needle (108) that extends into the blood collection holder (106).

7. The catheter and holder assembly (85) according to any one of claims 1 to 6, wherein the blood collection holder (106) comprises an interior support column (630) projecting proximally from the end wall (604) into the interior space (620), the interior support column (630) having the bore (632) passing therethrough that the needle is inserted through.

8. The catheter and holder assembly (85) according to any one of claims 1 to 7, wherein the blood collection holder (106) comprises mating flange (620) extending distally from an end wall (604), the distal projection (612) extending distally of the mating flange (620).

9. The catheter and holder assembly (85) according to any one of claims 1 to 8, wherein the needle (108) has a bend (109) corresponding to a bend (633) in the blood collection holder (106) for fitment.

10. The catheter and holder assembly (85) according to any one of claims 1 to 9, further comprising a needle guard (132) comprising a surface that is located to a side of the needle in the ready to use position and wherein the surface is moveable distal of the sharpened distal needle tip (110) in a protective position to cover the sharpened distal needle tip (110).

11. The catheter and holder assembly according to any one of claims 1 to 10, further comprising a side port (500) and a tubing (540) attached to the side port (500) or a valve (136) with a valve opener (134) located within the catheter hub (102).

12. A method of assembling a catheter and holder assembly (85), the method comprising:
mounting a needle (108), comprising a sharpened distal needle tip (110) and a sharpened proximal needle tip (200), to a blood collection holder (106);
coupling the blood collection holder (106) to a catheter hub (102) comprising a catheter tube (104) so that the needle (108) extends through the catheter hub (102) and the catheter tube (104) and the sharpened distal needle tip (110) is located distally of a distal end opening (112) of the catheter tube (104); and
wherein the blood collection holder (106) has a body 600 defining an interior space (620) that is dimensioned to receive a vacutainer and the sharpened proximal needle tip (200) is located within the interior space (620).

13. The method according to claim 12, wherein the blood collection holder (106) comprises an end wall (604) and a sidewall (607) defining the interior space (620) and a proximal opening.

14. The method according to claims 12 or 13, further comprising a flange (602) at the proximal end of the blood collection holder (106), the flange (602) comprising a surface for gripping.

15. The method according to any one of claims 12 to 14, further comprising:
coupling a deformable shield (252) in the interior space (620) of the blood collection holder (106), the deformable shield (252) covering the sharpened proximal needle tip (200).

16. The method according to any one of claims 12 to 15, wherein the blood collection holder (106) comprises a distal projection (612) extending distally from the end wall (604), the distal projection (612) projecting into an interior of the catheter hub (102).

17. The method according to any one of claims 12 to 16, wherein the blood collection holder (106) comprises a distal ring flange (170) arranged radially outward of the distal projection (612).

## Patentansprüche

1. Katheter- und Halteranordnung (85), umfassend:
einen Katheteransatz (102), der einen Katheterschlauch (104) mit einer distalen Öffnung (112), die an dem Katheteransatz (102) angebracht ist, aufweist;
eine Nadel (108), umfassend eine geschärfte distale Nadelspitze (110), die aus der distalen Öffnung (112) des Katheterschlauchs (104) herausragt; und
einen Blutsammelhalter (106), der ein Gehäuse (600) aufweist, das einen Innenraum (620), der bemessen ist, um einen Vacutainer aufzunehmen, aufweist;
**dadurch gekennzeichnet, dass** der Blutsammelhalter (106) einen Nadelansatz (106a) mit einer Bohrung (632), durch die die Nadel (108) verläuft, aufweist;
die Nadel (108) eine geschärfte proximale Nadelspitze (200) umfasst; und
der Katheteransatz (102) mit dem Blutsammelhalter (106) in Kontakt ist und sich die geschärfte proximale Nadelspitze (200) innerhalb des Innenraums (620) des Gehäuses (600) in einer gebrauchsfertigen Position befindet.

2. Katheter- und Halteranordnung (85) nach Anspruch 1, wobei die Nadel (108) eine Kerbe, eine Crimpung, eine Hülse oder eine Anhäufung, die sich proximal der geschärften distalen Nadelspitze (110) befinden, umfasst.

3. Katheter- und Halteranordnung (85) nach Anspruch 1 oder 2, wobei der Blutsammelhalter (106) eine Endwand (604) und eine Seitenwand (607), die den Innenraum (620) definieren, umfasst und der Nadelansatz (106a) mit der Endwand (604) integriert ist.

4. Katheter- und Halteranordnung (85) nach einem der Ansprüche 1 bis 3, wobei die geschärfte proximale Nadelspitze (200) in dem Innenraum (620) des Blutsammelhalters (106) ist, wenn die Katheter- und Halteranordnung in der gebrauchsfertigen Position ist.

5. Katheter- und Halteranordnung (85) nach einem der Ansprüche 1 bis 4, wobei der Blutsammelhalter (106) einen Vorsprung (612), der in der gebrauchsfertigen Position in den Katheteransatz (102) hineinragt, aufweist.

6. Katheter- und Halteranordnung (85) nach einem der Ansprüche 1 bis 5, wobei ein Abschnitt der Nadel (108), der sich distal des Blutsammelhalters (106) erstreckt, länger als ein Abschnitt der Nadel (108) ist, der sich in den Blutsammelhalter (106) hineinerstreckt.

7. Katheter- und Halteranordnung (85) nach einem der Ansprüche 1 bis 6, wobei der Blutsammelhalter (106) eine Innenstützsäule (630), die proximal von der Endwand (604) in den Innenraum (620) hineinragt, umfasst, wobei die Bohrung (632), durch die die Nadel eingeführt wird, durch die Innenstützsäule (630) verläuft.

8. Katheter- und Halteranordnung (85) nach einem der Ansprüche 1 bis 7, wobei der Blutsammelhalter (106) einen Gegenflansch (620), der sich distal von einer Endwand (604) erstreckt, umfasst, wobei sich der distale Vorsprung (612) distal des Gegenflansches (620) erstreckt.

9. Katheter- und Halteranordnung (85) nach einem der Ansprüche 1 bis 8, wobei die Nadel (108) eine Biegung (109), die einer Biegung (633) in dem Blutsammelhalter (106) entspricht, für Passung aufweist.

10. Katheter- und Halteranordnung (85) nach einem der Ansprüche 1 bis 9, ferner umfassend einen Nadelschutz (132), umfassend eine Oberfläche, die sich in der gebrauchsfertigen Position an einer Seite der Nadel befindet, und wobei die Oberfläche distal der geschärften distalen Nadelspitze (110) in einer Schutzposition bewegbar ist, um die geschärfte distale Nadelspitze (110) abzudecken.

11. Katheter- und Halteranordnung nach einem der Ansprüche 1 bis 10, ferner umfassend einen Seitenport (500) und eine Schlauchleitung (540), die an dem Seitenport (500) angebracht ist, oder ein Ventil (136) mit einem Ventilöffner (134), der sich innerhalb des Katheteransatzes (102) befindet.

12. Verfahren zum Zusammenbauen einer Katheter- und Halteranordnung (85), das Verfahren umfassend:
Montieren einer Nadel (108), umfassend eine geschärfte distale Nadelspitze (110) und eine geschärfte proximale Nadelspitze (200), an einem Blutsammelhalter (106);
Koppeln des Blutsammelhalters (106) mit einem Katheteransatz (102), umfassend einen Katheterschlauch (104), sodass sich die Nadel (108) durch den Katheteransatz (102) und den Katheterschlauch (104) erstreckt und sich die geschärfte distale Nadelspitze (110) distal einer distalen Endöffnung (112) des Katheterschlauchs (104) befindet; und
wobei der Blutsammelhalter (106) einen Körper 600, der einen Innenraum (620) definiert, der bemessen ist, um einen Vacutainer aufzunehmen, aufweist, und sich die geschärfte proximale Nadelspitze (200) innerhalb des Innenraums (620) befindet.

13. Verfahren nach Anspruch 12, wobei der Blutsammelhalter (106) eine Endwand (604) und eine Seitenwand (607), die den Innenraum (620) und eine proximale Öffnung definieren, umfasst.

14. Verfahren nach Anspruch 12 oder 13, ferner umfassend einen Flansch (602) an dem proximalen Ende des Blutsammelhalters (106), der Flansch (602) umfassend eine Oberfläche zum Greifen.

15. Verfahren nach einem der Ansprüche 12 bis 14, ferner umfassend:
Koppeln eines verformbaren Schilds (252) in dem Innenraum (620) des Blutsammelhalters (106), wobei der verformbare Schild (252) die geschärfte proximale Nadelspitze (200) abdeckt.

16. Verfahren nach einem der Ansprüche 12 bis 15, wobei der Blutsammelhalter (106) einen distalen Vorsprung (612), der sich distal von der Endwand (604) erstreckt, umfasst, wobei der distale Vorsprung (612) in ein Inneres des Katheteransatzes (102) hineinragt.

17. Verfahren nach einem der Ansprüche 12 bis 16, wobei der Blutsammelhalter (106) einen distalen Ringflansch (170), der radial außerhalb des distalen Vorsprungs (612) arrangiert ist, umfasst.

## Revendications

1. Ensemble cathéter et support (85) comprenant :
un embout de cathéter (102) ayant un tube de cathéter (104) avec une ouverture distale (112), fixé à l'embout de cathéter (102) ;
une aiguille (108) comprenant une pointe d'aiguille distale effilée (110) qui fait saillie de l'ouverture distale (112) du tube de cathéter (104) ; et
un support de collecte de sang (106) ayant un logement (600) ayant un espace intérieur (620) dimensionné pour recevoir un vacutainer ;
**caractérisé en ce que** le support de collecte de sang (106) a un connecteur d'aiguille (106a) avec un alésage (632) ayant l'aiguille (108) passant à travers celui-ci ;
l'aiguille (108) comprend une pointe d'aiguille proximale effilée (200) ; et
l'embout de cathéter (102) est en contact avec le support de collecte de sang (106) et la pointe d'aiguille proximale effilée (200) est localisée au sein de l'espace intérieur (620) du logement (600) dans une position prête à l'emploi.

2. Ensemble cathéter et support (85) selon la revendication 1, dans lequel l'aiguille (108) comprend une encoche, un sertissage, un manchon ou un habillage localisé proximalement par rapport à la pointe d'aiguille distale effilée (110).

3. Ensemble cathéter et support (85) selon les revendications 1 ou 2, dans lequel le support de collecte de sang (106) comprend une paroi d'extrémité (604) et une paroi latérale (607) définissant l'espace intérieur (620) et le connecteur d'aiguille (106a) est intégré à la paroi d'extrémité (604).

4. Ensemble cathéter et support (85) selon l'une quelconque des revendications 1 à 3, dans lequel la pointe d'aiguille proximale effilée (200) est dans l'espace intérieur (620) du support de collecte de sang (106) lorsque l'ensemble cathéter et support est dans la position prête à l'emploi.

5. Ensemble cathéter et support (85) selon l'une quelconque des revendications 1 à 4, dans lequel le support de collecte de sang (106) a une saillie (612) qui fait saillie dans l'embout de cathéter (102) dans la position prête à l'emploi.

6. Ensemble cathéter et support (85) selon l'une quelconque des revendications 1 à 5, dans lequel une partie de l'aiguille (108) qui s'étend distalement par rapport au support de collecte de sang (106) est plus longue qu'une partie de l'aiguille (108) qui s'étend dans le support de collecte de sang (106).

7. Ensemble cathéter et support (85) selon l'une quelconque des revendications 1 à 6, dans lequel le support de collecte de sang (106) comprend une colonne de support intérieure (630) faisant saillie proximalement à partir de la paroi d'extrémité (604) dans l'espace intérieur (620), la colonne de support intérieure (630) ayant l'alésage (632) la traversant à travers lequel l'aiguille est insérée.

8. Ensemble cathéter et support (85) selon l'une quelconque des revendications 1 à 7, dans lequel le support de collecte de sang (106) comprend une contre-bride (620) s'étendant distalement à partir d'une paroi d'extrémité (604), la saillie distale (612) s'étendant distalement par rapport à la contre-bride (620).

9. Ensemble cathéter et support (85) selon l'une quelconque des revendications 1 à 8, dans lequel l'aiguille (108) a une courbure (109) correspondant à une courbure (633) dans le support de collecte de sang (106) pour ajustement.

10. Ensemble cathéter et support (85) selon l'une quelconque des revendications 1 à 9, comprenant en outre une protection d'aiguille (132) comprenant une surface qui est localisée sur un côté de l'aiguille dans la position prête à l'emploi et dans lequel la surface peut être déplacée distalement par rapport à la pointe d'aiguille distale effilée (110) dans une position de protection pour couvrir la pointe d'aiguille distale effilée (110).

11. Ensemble cathéter et support selon l'une quelconque des revendications 1 à 10, comprenant en outre un orifice latéral (500) et un tubage (540) fixé à l'orifice latéral (500) ou une valve (136) avec un système d'ouverture de valve (134) localisé au sein de l'embout de cathéter (102).

12. Procédé d'assemblage d'un ensemble cathéter et support (85), le procédé comprenant :
le montage d'une aiguille (108), comprenant une pointe d'aiguille distale effilée (110) et une pointe d'aiguille proximale effilée (200), à un support de collecte de sang (106) ;
l'accouplement du support de collecte de sang (106) à un embout de cathéter (102) comprenant un tube de cathéter (104) de sorte que l'aiguille (108) s'étend à travers l'embout de cathéter (102) et le tube de cathéter (104) et la pointe d'aiguille distale effilée (110) est localisée distalement par rapport à une ouverture d'extrémité distale (112) du tube de cathéter (104) ; et
dans lequel le support de collecte de sang (106) a un corps 600 définissant un espace intérieur (620) qui est dimensionné pour recevoir un vacutainer et la pointe d'aiguille proximale effilée (200) est localisée au sein de l'espace intérieur (620).

13. Procédé selon la revendication 12, dans lequel le support de collecte de sang (106) comprend une paroi d'extrémité (604) et une paroi latérale (607) définissant l'espace intérieur (620) et une ouverture proximale.

14. Procédé selon les revendications 12 ou 13, comprenant en outre une bride (602) au niveau de l'extrémité proximale du support de collecte de sang (106), la bride (602) comprenant une surface permettant de saisir.

15. Procédé selon l'une quelconque des revendications 12 à 14, comprenant en outre :
l'accouplement d'une protection déformable (252) dans l'espace intérieur (620) du support de collecte de sang (106), la protection déformable (252) couvrant la pointe d'aiguille proximale effilée (200).

16. Procédé selon l'une quelconque des revendications 12 à 15, dans lequel le support de collecte de sang (106) comprend une saillie distale (612) s'étendant distalement à partir de la paroi d'extrémité (604), la saillie distale (612) faisant saillie dans un intérieur de l'embout de cathéter (102).

17. Procédé selon l'une quelconque des revendications 12 à 16, dans lequel le support de collecte de sang (106) comprend une bride annulaire distale (170) agencée radialement vers l'extérieur de la saillie distale (612).
